# EUROPEAN PATENT SPECIFICATION

(11) **EP 2 093 283 B1**
(45) Date of publication and mention of the grant of the patent: **11.04.2012**
(21) Application number: 08151759.1
(22) Date of filing: 21.02.2008
(51) Int. Cl.: C12N 9/04, C12N 9/08, C12N 9/10, C12N 15/82, C12N 5/04, A01H 5/00, A01H 5/10

(54) **Means for improving agrobiological traits in a plant by providing a plant cell comprising in its chloroplasts enzymatic activities for converting glycolate into malate**
Mittel zur Verbesserung von agrobiologischen Eigenschaften einer Pflanze durch Bereitstellen einer Pflanzenzelle mit chloroplastischen Enzymaktivitäten zum Umwandeln von Glycolat in Malat
Moyens pour améliorer les caractéristiques agrobiologiques dans une plante en fournissant une cellule végétale contenant dans ses chloroplastes des activités enzymatiques pour la conversion de glycolate en malate

(43) Date of publication of application: 26.08.2009
(73) Proprietor: UNIVERSITÄT ZU KÖLN, 50923 Köln (DE)
(72) Inventor: Maurino, Verónica G., 50939 Köln (DE); Flügge, Ulf-Ingo, 50997 Köln (DE)
(74) Representative: Dick, Alexander

(56) References cited:
- WO-A-03/100066
- BOLDT RALF ET AL: "Decrease in glycolate pathway enzyme activities in plastids and peroxisomes of the albostrians mutant of barley (Hordeum vulgare L.)" PLANT SCIENCE (SHANNON), vol. 124, no. 1, 1997, pages 33-40, XP002489620 ISSN: 0168-9452

### SEQUENCE LISTING

<110> Universität zu Köln, Zentrum fur Molekularbiologische Medizin
<120> Means for improving agrobiological traits in a plant by providing a plant cell comprising in its chloroplasts enzymatic activities for converting glycolate into malate
<130> BPS65361EP
<160> 18
<170> PatentIn version 3.4
<210> 1
   <211> 1104
   <212> DNA
   <213> A. thaliana
<400> 1
   atggagatca ctaacgttac cgagtatgat gcaatcgcaa agcagaagct gcctaagatg     60
   gtgtacgact actatgcatc tggtgcagaa gaccaatgga ctcttcaaga gaacagaaac    120
   gcttttgcaa ggatcctctt tcggcctcgg attctgattg atgtgagcaa gattgacatg    180
   acaaccaccg tcttggggtt caagatctcg atgcccatca tggttgctcc aactgccatg    240
   caaaagatgg ctcaccctga tggggaatat gctactgcta gagctgcatc tgcagctgga    300
   actatcatga cactatcttc atgggctact tccagcgttg aagaagttgc gtctacaggg    360
   ccagggatcc gattcttcca gctctatgta tacaagaaca ggaatgtggt tgagcagctc    420
   gtgagaagag ctgagagggc tgggttcaaa gccattgctc tcactgtaga caccccaagg    480
   ctaggccgca gagagtctga tatcaagaac agattcactt tgcctccaaa cctgacattg    540
   aagaactttg aaggacttga cctcggaaag atggacgagg ccaatgactc tggcttggct    600
   tcatatgttg ctggtcaaat tgaccgtacc ttaagctgga aggatgtcca gtggctccag    660
   acaatcacca agttgcccat tcttgtcaaa ggtgttctta caggagagga tgcaaggata    720
   gcgattcaag ctggtgcagc cggaatcatt gtatcaaacc atggagctcg ccagcttgac    780
   tatgtcccag caaccatctc ggcccttgaa gaggttgtca aagcgacaca aggacgaatt    840
   cctgtcttct tggatggtgg tgttcgacgt ggcactgatg tcttcaaagc acttgcactt    900
   ggagcctccg ggatatttat tggaagacca gtggtattct cattggcagc tgaaggagag    960
   gctggagtta gaaaggtgct tcaaatgcta cgtgatgagt tcgagctgac catggcactg   1020
   agtgggtgtc ggtccctaaa ggaaatctcc cgtaaccaca ttaccaccga atgggacact   1080
   ccacgtcctt cagccaggtt atag                                          1104
<210> 2
   <211> 367
   <212> PRT
   <213> A. thaliana
<400> 2
   Met Glu Ile Thr Asn Val Thr Glu Tyr Asp Ala Ile Ala Lys Gln Lys
   1               5                   10                  15
   Leu Pro Lys Met Val Tyr Asp Tyr Tyr Ala Ser Gly Ala Glu Asp Gln
               20                  25                  30
   Trp Thr Leu Gln Glu Asn Arg Asn Ala Phe Ala Arg Ile Leu Phe Arg
           35                  40                  45
   Pro Arg Ile Leu Ile Asp Val Ser Lys Ile Asp Met Thr Thr Thr Val
       50                  55                  60
   Leu Gly Phe Lys Ile Ser Met Pro Ile Met Val Ala Pro Thr Ala Met
   65                  70                  75                  80
   Gln Lys Met Ala His Pro Asp Gly Glu Tyr Ala Thr Ala Arg Ala Ala
                   85                  90                  95
   Ser Ala Ala Gly Thr Ile Met Thr Leu Ser Ser Trp Ala Thr Ser Ser
               100                 105                 110
   Val Glu Glu Val Ala Ser Thr Gly Pro Gly Ile Arg Phe Phe Gln Leu
           115                 120                 125
   Tyr Val Tyr Lys Asn Arg Asn Val Val Glu Gln Leu Val Arg Arg Ala
       130                 135                 140
   Glu Arg Ala Gly Phe Lys Ala Ile Ala Leu Thr Val Asp Thr Pro Arg
   145                 150                 155                 160
   Leu Gly Arg Arg Glu Ser Asp Ile Lys Asn Arg Phe Thr Leu Pro Pro
                   165                 170                 175
   Asn Leu Thr Leu Lys Asn Phe Glu Gly Leu Asp Leu Gly Lys Met Asp
               180                 185                 190
   Glu Ala Asn Asp Ser Gly Leu Ala Ser Tyr Val Ala Gly Gln Ile Asp
           195                 200                 205
   Arg Thr Leu Ser Trp Lys Asp Val Gln Trp Leu Gln Thr Ile Thr Lys
       210                 215                 220
   Leu Pro Ile Leu Val Lys Gly Val Leu Thr Gly Glu Asp Ala Arg Ile
   225                 230                 235                 240
   Ala Ile Gln Ala Gly Ala Ala Gly Ile Ile Val Ser Asn His Gly Ala
                   245                 250                 255
   Arg Gln Leu Asp Tyr Val Pro Ala Thr Ile Ser Ala Leu Glu Glu Val
               260                 265                 270
   Val Lys Ala Thr Gln Gly Arg Ile Pro Val Phe Leu Asp Gly Gly Val
           275                 280                 285
   Arg Arg Gly Thr Asp Val Phe Lys Ala Leu Ala Leu Gly Ala Ser Gly
       290                 295                 300
   Ile Phe Ile Gly Arg Pro Val Val Phe Ser Leu Ala Ala Glu Gly Glu
   305                 310                 315                 320
   Ala Gly Val Arg Lys Val Leu Gln Met Leu Arg Asp Glu Phe Glu Leu
                   325                 330                 335
   Thr Met Ala Leu Ser Gly Cys Arg Ser Leu Lys Glu Ile Ser Arg Asn
               340                 345                 350
   His Ile Thr Thr Glu Trp Asp Thr Pro Arg Pro Ser Ala Arg Leu
           355                 360                 365
<210> 3
   <211> 1947
   <212> DNA
   <213> Cucurbita pepo
<400> 3
   atgggatcgc tgggaatgta ttctgaatcg gcagtaagga agaaaagtag ccgaggctac    60
   gatgttccag agggagtgga cattcgggga cgttatgatg aagaatttgc caggattctc   120
   aacaaggaag ccttgctgtt tgtggctgat ttacagagga ctttcagaaa ccacataagg   180
   tattcgatgg aatgccgcag agaagccaaa aggaggtaca atgaaggggc ggtgccgggg   240
   tttgatccgg cgaccaagta tataagggaa tctgagtgga catgtgcatc agtccccccg   300
   gcagttgctg atcggagagt ggagatcacc ggacctgtgg agcggaagat gatcatcaac   360
   gcactcaatt ctggagctaa agttttcatg gcggactttg aagatgcact atcaccaaac   420
   tgggagaatt tgatgagggg gcaaattaat ctgaaggatg cagttgatgg gactataagc   480
   ttccatgaca aagctagaaa caaggtttat aaactgaacg atcagacagc caagctcttt   540
   gttcgccctc gaggttggca cttcgctgag gctcatatct tcatcgacgg cgagcctgcc   600
   accggctgtc ttgtggactt cgggctctac ttttttcaca accatgccaa tttccggcgc    660
   tctcaaggtc aaggttctgg ccctttcttt taccttccca aaatggagca ctccagggaa   720
   gcaaaaatat ggaacagtgt atttgagaga gcagagaaga tggcagggat agagaggggc   780
   agcatcaggg ccactgtgct gattgaaaca cttccagcag tgtttcaaat ggatgaaata   840
   ctctatgagc tgagggatca ttctgtggga ttgaactgtg gtagatggga ttacatattc   900
   agctatgtca agaccttcca ggctcaccta gatcgcctgt tacccgaccg agtccaagtc   960
   ggtatggcac aacatttcat gaggagttat tctgatctcc ttatcaggac ttgtcacacg  1020
   gtggtgtgcc acgtgggagg catggctgct caaattccaa ttagagacga cccgaaggca  1080
   aatgagatgg cacttgagct agtgaggaag gacaaattga gagaggcaaa ggcaggacat  1140
   gatggaacat gggcagcaca tccaggatta atcccagcat gtatggaagt cttcaccaac  1200
   agcatgggaa atgcccccaa tcagatccga tctgcaagac gagacgatgc tgcaaaccta  1260
   actgaagacg acctcttgca gcaaccgagg ggtgttcgta cattggaagg gctccggttg  1320
   aacacccgag tcggaattca gtacctagca gcatggctaa ccgggacagg ctctgtgcct  1380
   ctctacaacc ttatggaaga tgcagccaca gctgaaatca gcagggttca aaactggcaa  1440
   tggctgaagt atggagtgga attggatgga gatgggcttg gagtgagagt gaacaaggaa  1500
   ctgttcgcaa gagtggtgga agaagaaatg gaaaggattg aaagagaagt ggggaaggag  1560
   aaattcagga agggaatgta caaagaggct tgcaagatgt tcacaaggca atgcacagcg  1620
   ccaaccttgg atgattttct gaccttggat gcgtacaacc acatagtcat acatcatccc  1680
   agggagctgt ccaggctctg aaaaccgcac caaattggcc ttctttcgac aggtaagagt  1740
   tcttttgctt cccttctctt ctcttttctt ctcttccttg ttgctgagaa taacgtattt  1800
   gtattgtaat ttaagactct gtttgtttgt ccgtttcttt ttttaagctg tttgtgtcgg  1860
   ttatgtcaag aaattcagca attgcaagca aattggaact ctctccatga acctaaaaaa  1920
   aaaaaaaaaa aaaaaaaaaa aaaaaaa                                      1947
<210> 4
   <211> 566
   <212> PRT
   <213> Cucurbita pepo
<400> 4
   Met Gly Ser Leu Gly Met Tyr Ser Glu Ser Ala Val Arg Lys Lys Ser
   1               5                   10                  15
   Ser Arg Gly Tyr Asp Val Pro Glu Gly Val Asp Ile Arg Gly Arg Tyr
               20                  25                  30
   Asp Glu Glu Phe Ala Arg Ile Leu Asn Lys Glu Ala Leu Leu Phe Val
           35                  40                  45
   Ala Asp Leu Gln Arg Thr Phe Arg Asn His Ile Arg Tyr Ser Met Glu
       50                  55                  60
   Cys Arg Arg Glu Ala Lys Arg Arg Tyr Asn Glu Gly Ala Val Pro Gly
   65                  70                  75                  80
   Phe Asp Pro Ala Thr Lys Tyr Ile Arg Glu Ser Glu Trp Thr Cys Ala
                   85                  90                  95
   Ser Val Pro Pro Ala Val Ala Asp Arg Arg Val Glu Ile Thr Gly Pro
               100                 105                 110
   Val Glu Arg Lys Met Ile Ile Asn Ala Leu Asn Ser Gly Ala Lys Val
           115                 120                 125
   Phe Met Ala Asp Phe Glu Asp Ala Leu Ser Pro Asn Trp Glu Asn Leu
       130                 135                 140
   Met Arg Gly Gln Ile Asn Leu Lys Asp Ala Val Asp Gly Thr Ile Ser
   145 150 155 160
   Phe His Asp Lys Ala Arg Asn Lys Val Tyr Lys Leu Asn Asp Gln Thr
   165 170 175
   Ala Lys Leu Phe Val Arg Pro Arg Gly Trp His Phe Ala Glu Ala His
   180 185 190
   Ile Phe Ile Asp Gly Glu Pro Ala Thr Gly Cys Leu Val Asp Phe Gly
   195 200 205
   Leu Tyr Phe Phe His Asn His Ala Asn Phe Arg Arg Ser Gln Gly Gln
   210 215 220
   Gly Ser Gly Pro Phe Phe Tyr Leu Pro Lys Met Glu His Ser Arg Glu
   225 230 235 240
   Ala Lys Ile Trp Asn Ser Val Phe Glu Arg Ala Glu Lys Met Ala Gly
   245 250 255
   Ile Glu Arg Gly Ser Ile Arg Ala Thr Val Leu Ile Glu Thr Leu Pro
   260 265 270
   Ala Val Phe Gln Met Asp Glu Ile Leu Tyr Glu Leu Arg Asp His Ser
   275 280 285
   Val Gly Leu Asn Cys Gly Arg Trp Asp Tyr Ile Phe Ser Tyr Val Lys
   290 295 300
   Thr Phe Gln Ala His Leu Asp Arg Leu Leu Pro Asp Arg Val Gln Val
   305 310 315 320
   Gly Met Ala Gln His Phe Met Arg Ser Tyr Ser Asp Leu Leu Ile Arg
   325 330 335
   Thr Cys His Thr Val Val Cys His Val Gly Gly Met Ala Ala Gln Ile
   340 345 350
   Pro Ile Arg Asp Asp Pro Lys Ala Asn Glu Met Ala Leu Glu Leu Val
   355 360 365
   Arg Lys Asp Lys Leu Arg Glu Ala Lys Ala Gly His Asp Gly Thr Trp
   370 375 380
   Ala Ala His Pro Gly Leu Ile Pro Ala Cys Met Glu Val Phe Thr Asn
   385 390 395 400
   Ser Met Gly Asn Ala Pro Asn Gln Ile Arg Ser Ala Arg Arg Asp Asp
   405 410 415
   Ala Ala Asn Leu Thr Glu Asp Asp Leu Leu Gln Gln Pro Arg Gly Val
   420 425 430
   Arg Thr Leu Glu Gly Leu Arg Leu Asn Thr Arg Val Gly Ile Gln Tyr
           435                 440                 445
   Leu Ala Ala Trp Leu Thr Gly Thr Gly Ser Val Pro Leu Tyr Asn Leu
       450                 455                 460
   Met Glu Asp Ala Ala Thr Ala Glu Ile Ser Arg Val Gln Asn Trp Gln
   465                 470                 475                 480
   Trp Leu Lys Tyr Gly Val Glu Leu Asp Gly Asp Gly Leu Gly Val Arg
                   485                 490                 495
   Val Asn Lys Glu Leu Phe Ala Arg Val Val Glu Glu Glu Met Glu Arg
               500                 505                 510
   Ile Glu Arg Glu Val Gly Lys Glu Lys Phe Arg Lys Gly Met Tyr Lys
           515                 520                 525
   Glu Ala Cys Lys Met Phe Thr Arg Gln Cys Thr Ala Pro Thr Leu Asp
       530                 535                 540
   Asp Phe Leu Thr Leu Asp Ala Tyr Asn His Ile Val Ile His His Pro
   545                 550                 555                 560
   Arg Glu Leu Ser Arg Leu
                   565
<210> 5
   <211> 2262
   <212> DNA
   <213> Escherichia coli
<400> 5
   atgtcgcaac ataacgaaaa gaacccacat cagcaccagt caccactaca cgattccagc 60
   gaagcgaaac cggggatgga ctcactggca cctgaggacg gctctcatcg tccagcggct 120
   gaaccaacac cgccaggtgc acaacctacc gccccaggga gcctgaaagc ccctgatacg 180
   cgtaacgaaa aacttaattc tctggaagac gtacgcaaag gcagtgaaaa ttatgcgctg 240
   accactaatc agggcgtgcg catcgccgac gatcaaaact cactgcgtgc cggtagccgt 300
   ggtccaacgc tgctggaaga ttttattctg cgcgagaaaa tcacccactt tgaccatgag 360
   cgcattccgg aacgtattgt tcatgcacgc ggatcagccg ctcacggtta tttccagcca 420
   tataaaagct taagcgatat taccaaagcg gatttcctct cagatccgaa caaaatcacc 480
   ccagtatttg tacgtttctc taccgttcag ggtggtgctg gctctgctga taccgtgcgt 540
   gatatccgtg gctttgccac caagttctat accgaagagg gtatttttga cttcgttggc 600
   aataacacgc caatcttctt tatccaggat gcgcataaat tccccgattt tgttcatgcg 660
   gtaaaaccag aaccgcactg ggcaattcca caagggcaaa gtgcccacga tactttctgg 720
   gattatgttt ctctgcaacc tgaaactctg cacaacgtga tgtgggcgat gtcggatcgc 780
   ggcatccccc gcagttaccg caccatggaa ggcttcggta ttcacacctt ccgcctgatt 840
   aatgccgaag ggaaggcaac gtttgtacgt ttccactgga aaccactggc aggtaaagcc 900
   tcactcgttt gggatgaagc acaaaaactc accggacgtg acccggactt ccaccgccgc 960
   gagttgtggg aagccattga agcaggcgat tttccggaat acgaactggg cttccagttg 1020
   attcctgaag aagatgaatt caagttcgac ttcgatcttc tcgatccaac caaacttatc 1080
   ccggaagaac tggtgcccgt tcagcgtgtc ggcaaaatgg tgctcaatcg caacccggat 1140
   aacttctttg ctgaaaacga acaggcggct ttccatcctg ggcatatcgt gccgggactg 1200
   gacttcacca acgatccgct gttgcaggga cgtttgttct cctataccga tacacaaatc 1260
   agtcgtcttg gtgggccgaa tttccatgag attccgatta accgtccgac ctgcccttac 1320
   cataatttcc agcgtgacgg catgcatcgc atggggatcg acactaaccc ggcgaattac 1380
   gaaccgaact cgattaacga taactggccg cgcgaaacac cgccggggcc gaaacgcggc 1440
   ggttttgaat cataccagga gcgcgtggaa ggcaataaag ttcgcgagcg cagcccatcg 1500
   tttggcgaat attattccca tccgcgtctg ttctggctaa gtcagacgcc atttgagcag 1560
   cgccatattg tcgatggttt cagttttgag ttaagcaaag tcgttcgtcc gtatattcgt 1620
   gagcgcgttg ttgaccagct ggcgcatatt gatctcactc tggcccaggc ggtggcgaaa 1680
   aatctcggta tcgaactgac tgacgaccag ctgaatatca ccccacctcc ggacgtcaac 1740
   ggtctgaaaa aggatccatc cttaagtttg tacgccattc ctgacggtga tgtgaaaggt 1800
   cgcgtggtag cgattttact taatgatgaa gtgagatcgg cagaccttct ggccattctc 1860
   aaggcgctga aggccaaagg cgttcatgcc aaactgctct actcccgaat gggtgaagtg 1920
   actgcggatg acggtacggt gttgcctata gccgctacct ttgccggtgc accttcgctg 1980
   acggtcgatg cggtcattgt cccttgcggc aatatcgcgg atatcgctga caacggcgat 2040
   gccaactact acctgatgga agcctacaaa caccttaaac cgattgcgct ggcgggtgac 2100
   gcgcgcaagt ttaaagcaac aatcaagatc gctgaccagg gtgaagaagg gattgtggaa 2160
   gctgacagcg ctgacggtag ttttatggat gaactgctaa cgctgatggc agcacaccgc 2220
   gtgtggtcac gcattcctaa gattgacaaa attcctgcct ga 2262
<210> 6
   <211> 753
   <212> PRT
   <213> Escherichia coli
<400> 6
   Met Ser Gln His Asn Glu Lys Asn Pro His Gln His Gln Ser Pro Leu
   1               5                   10                  15
   His Asp Ser Ser Glu Ala Lys Pro Gly Met Asp Ser Leu Ala Pro Glu
               20                  25                  30
   Asp Gly Ser His Arg Pro Ala Ala Glu Pro Thr Pro Pro Gly Ala Gln
           35                  40                  45
   Pro Thr Ala Pro Gly Ser Leu Lys Ala Pro Asp Thr Arg Asn Glu Lys
       50                  55                  60
   Leu Asn Ser Leu Glu Asp Val Arg Lys Gly Ser Glu Asn Tyr Ala Leu
   65                  70                  75                  80
   Thr Thr Asn Gln Gly Val Arg Ile Ala Asp Asp Gln Asn Ser Leu Arg
                   85                  90                  95
   Ala Gly Ser Arg Gly Pro Thr Leu Leu Glu Asp Phe Ile Leu Arg Glu
               100                 105                 110
   Lys Ile Thr His Phe Asp His Glu Arg Ile Pro Glu Arg Ile Val His
           115                 120                 125
   Ala Arg Gly Ser Ala Ala His Gly Tyr Phe Gln Pro Tyr Lys Ser Leu
       130                 135                 140
   Ser Asp Ile Thr Lys Ala Asp Phe Leu Ser Asp Pro Asn Lys Ile Thr
   145                 150                 155                 160
   Pro Val Phe Val Arg Phe Ser Thr Val Gln Gly Gly Ala Gly Ser Ala
                   165                 170                 175
   Asp Thr Val Arg Asp Ile Arg Gly Phe Ala Thr Lys Phe Tyr Thr Glu
               180                 185                 190
   Glu Gly Ile Phe Asp Leu Val Gly Asn Asn Thr Pro Ile Phe Phe Ile
           195                 200                 205
   Gln Asp Ala His Lys Phe Pro Asp Phe Val His Ala Val Lys Pro Glu
       210                 215                 220
   Pro His Trp Ala Ile Pro Gln Gly Gln Ser Ala His Asp Thr Phe Trp
   225                 230                 235                 240
   Asp Tyr Val Ser Leu Gln Pro Glu Thr Leu His Asn Val Met Trp Ala
                   245                 250                 255
   Met Ser Asp Arg Gly Ile Pro Arg Ser Tyr Arg Thr Met Glu Gly Phe
               260                 265                 270
   Gly Ile His Thr Phe Arg Leu Ile Asn Ala Glu Gly Lys Ala Thr Phe
           275                 280                 285
   Val Arg Phe His Trp Lys Pro Leu Ala Gly Lys Ala Ser Leu Val Trp
       290                 295                 300
   Asp Glu Ala Gln Lys Leu Thr Gly Arg Asp Pro Asp Phe His Arg Arg
   305                 310                 315                 320
   Glu Leu Trp Glu Ala Ile Glu Ala Gly Asp Phe Pro Glu Tyr Glu Leu
                   325                 330                 335
   Gly Phe Gln Leu Ile Pro Glu Glu Asp Glu Phe Lys Phe Asp Phe Asp
               340                 345                 350
   Leu Leu Asp Pro Thr Lys Leu Ile Pro Glu Glu Leu Val Pro Val Gln
           355                 360                 365
   Arg Val Gly Lys Met Val Leu Asn Arg Asn Pro Asp Asn Phe Phe Ala
       370                 375                 380
   Glu Asn Glu Gln Ala Ala Phe His Pro Gly His Ile Val Pro Gly Leu
   385                 390                 395                 400
   Asp Phe Thr Asn Asp Pro Leu Leu Gln Gly Arg Leu Phe Ser Tyr Thr
                   405                 410                 415
   Asp Thr Gln Ile Ser Arg Leu Gly Gly Pro Asn Phe His Glu Ile Pro
               420                 425                 430
   Ile Asn Arg Pro Thr Cys Pro Tyr His Asn Phe Gln Arg Asp Gly Met
           435                 440                 445
   His Arg Met Gly Ile Asp Thr Asn Pro Ala Asn Tyr Glu Pro Asn Ser
       450                 455                 460
   Ile Asn Asp Asn Trp Pro Arg Glu Thr Pro Pro Gly Pro Lys Arg Gly
   465                 470                 475                 480
   Gly Phe Glu Ser Tyr Gln Glu Arg Val Glu Gly Asn Lys Val Arg Glu
                   485                 490                 495
   Arg Ser Pro Ser Phe Gly Glu Tyr Tyr Ser His Pro Arg Leu Phe Trp
               500                 505                 510
   Leu Ser Gln Thr Pro Phe Glu Gln Arg His Ile Val Asp Gly Phe Ser
           515                 520                 525
   Phe Glu Leu Ser Lys Val Val Arg Pro Tyr Ile Arg Glu Arg Val Val
       530                 535                 540
   Asp Gln Leu Ala His Ile Asp Leu Thr Leu Ala Gln Ala Val Ala Lys
   545                 550                 555                 560
   Asn Leu Gly Ile Glu Leu Thr Asp Asp Gln Leu Asn Ile Thr Pro Pro
                   565                 570                 575
   Pro Asp Val Asn Gly Leu Lys Lys Asp Pro Ser Leu Ser Leu Tyr Ala
               580                 585                 590
   Ile Pro Asp Gly Asp Val Lys Gly Arg Val Val Ala Ile Leu Leu Asn
           595                 600                 605
   Asp Glu Val Arg Ser Ala Asp Leu Leu Ala Ile Leu Lys Ala Leu Lys
       610                 615                 620
   Ala Lys Gly Val His Ala Lys Leu Leu Tyr Ser Arg Met Gly Glu Val
   625                 630                 635                 640
   Thr Ala Asp Asp Gly Thr Val Leu Pro Ile Ala Ala Thr Phe Ala Gly
                   645                 650                 655
   Ala Pro Ser Leu Thr Val Asp Ala Val Ile Val Pro Cys Gly Asn Ile
               660                 665                 670
   Ala Asp Ile Ala Asp Asn Gly Asp Ala Asn Tyr Tyr Leu Met Glu Ala
           675                 680                 685
   Tyr Lys His Leu Lys Pro Ile Ala Leu Ala Gly Asp Ala Arg Lys Phe
       690                 695                 700
   Lys Ala Thr Ile Lys Ile Ala Asp Gln Gly Glu Glu Gly Ile Val Glu
   705                 710                 715                 720
   Ala Asp Ser Ala Asp Gly Ser Phe Met Asp Glu Leu Leu Thr Leu Met
                   725                 730                 735
   Ala Ala His Arg Val Trp Ser Arg Ile Pro Lys Ile Asp Lys Ile Pro
               740                 745                 750
   Ala
<210> 7
   <211> 1704
   <212> DNA
   <213> A. thalia
<400> 7
   atggctttcg cttcaaaatt cgctcgttct aaaactattc tctcttttct ccggccttgt 60
   cgtcagctcc actcgacgcc caagtcaacc ggtgacgtga ccgtactttc tccggtgaag 120
   ggacgccgga gacttccaac ttgctggtcg agctctctgt tcccattggc gatagctgcc 180
   tccgccactt ctttcgctta cctgaatctg tcgaatcctt ctatcagtga atcatcatcg 240
   gctttggatt caagagatat aactgttggt ggaaaagata gcactgaagc tgttgttaaa 300
   ggagaataca aacaagtccc taaggagctt atttctcaat tgaaaactat cctcgaggat 360
   aacttgacaa ctgactacga tgagaggtac ttccatggga agccccagaa cagttttcac 420
   aaggcagtca acattcctga tgtcgttgtt ttccctaggt ccgaagaaga agtctccaag 480
   attcttaaat cctgcaatga atataaggtt cctattgtac catatggtgg ggcaacatcg    540
   atcgagggtc atacccttgc tccaaaagga ggtgtgtgca ttgacatgtc attaatgaag    600
   agggtgaaag cattacatgt ggaggatatg gatgttattg ttgagcctgg aattggttgg 660
   ctggagctta atgaatattt ggaagagtat ggtctattct ttcctcttga tccaggacct 720
   ggtgcctcca taggaggcat gtgtgctacg cgttgctctg gctctttagc tgtaaggtat 780
   ggaactatgc gtgacaatgt tataagcctc aaggtggttc ttcctaatgg agatgttgtg 840
   aagacagctt cacgtgccag aaagagtgct gctggatacg atttgactcg cttgataatt 900
   gggagtgagg gtactttagg agtcattact gagattactc tccgacttca gaaaatccca 960
   cagcattcag tggtggcagt ttgcaatttc cctacagtta aggatgctgc agacgtggcc 1020
   attgccacta tgatgtctgg aatacaggtg tcaagagtgg aactccttga cgaggttcaa 1080
   atcagagcta ttaatatggc aaacgggaaa aatttgactg aagctccaac tctgatgttc 1140
   gagtttatag gaacagaggc atatacacgt gagcagacgc aaattgttca gcaaattgct 1200
   tccaaacaca atggatcaga ctttatgttc gcagaagaac ctgaagcaaa aaaagaactc 1260
   tggaagataa gaaaagaggc gctgtgggct tgctatgcta tggcgccagg tcatgaagca 1320
   atgattacag atgtctgtgt ccctttatct caccttgcag aactcatatc aagatccaaa 1380
   aaagagcttg atgcatcatc gttgttgtgt accgttattg ctcatgccgg agatggaaac 1440
   tttcacacat gtattatgtt tgatccaagc agtgaagagc agagaagaga agcagaaaga 1500
   ctgaaccact ttatggttca cagtgcactg tccatggatg gaacatgtac tggagaacac 1560
   ggtgttggaa caggaaaaat gaagtatctg gagaaggaac tgggaataga agcactgcaa 1620
   actatgaaga gaatcaagaa aacgttggac ccaaacgata tcatgaaccc gggaaagtta 1680
   attcctcctc atgtatgttt ctaa 1704
<210> 8
   <211> 567
   <212> PRT
   <213> A. thalia
<400> 8
   Met Ala Phe Ala Ser Lys Phe Ala Arg Ser Lys Thr Ile Leu Ser Phe
   1               5                   10                  15
   Leu Arg Pro Cys Arg Gln Leu His Ser Thr Pro Lys Ser Thr Gly Asp
               20                  25                  30
   Val Thr Val Leu Ser Pro Val Lys Gly Arg Arg Arg Leu Pro Thr Cys
           35                  40                  45
   Trp Ser Ser Ser Leu Phe Pro Leu Ala Ile Ala Ala Ser Ala Thr Ser
       50                  55                  60
   Phe Ala Tyr Leu Asn Leu Ser Asn Pro Ser Ile Ser Glu Ser Ser Ser
   65                  70                  75                  80
   Ala Leu Asp Ser Arg Asp Ile Thr Val Gly Gly Lys Asp Ser Thr Glu
                   85                  90                  95
   Ala Val Val Lys Gly Glu Tyr Lys Gln Val Pro Lys Glu Leu Ile Ser
               100                 105                 110
   Gln Leu Lys Thr Ile Leu Glu Asp Asn Leu Thr Thr Asp Tyr Asp Glu
           115                 120                 125
   Arg Tyr Phe His Gly Lys Pro Gln Asn Ser Phe His Lys Ala Val Asn
       130                 135                 140
   Ile Pro Asp Val Val Val Phe Pro Arg Ser Glu Glu Glu Val Ser Lys
   145                 150                 155                 160
   Ile Leu Lys Ser Cys Asn Glu Tyr Lys Val Pro Ile Val Pro Tyr Gly
                   165                 170                 175
   Gly Ala Thr Ser Ile Glu Gly His Thr Leu Ala Pro Lys Gly Gly Val
               180                 185                 190
   Cys Ile Asp Met Ser Leu Met Lys Arg Val Lys Ala Leu His Val Glu
           195                 200                 205
   Asp Met Asp Val Ile Val Glu Pro Gly Ile Gly Trp Leu Glu Leu Asn
       210                 215                 220
   Glu Tyr Leu Glu Glu Tyr Gly Leu Phe Phe Pro Leu Asp Pro Gly Pro
   225                 230                 235                 240
   Gly Ala Ser Ile Gly Gly Met Cys Ala Thr Arg Cys Ser Gly Ser Leu
                   245                 250                 255
   Ala Val Arg Tyr Gly Thr Met Arg Asp Asn Val Ile Ser Leu Lys Val
               260                 265                 270
   Val Leu Pro Asn Gly Asp Val Val Lys Thr Ala Ser Arg Ala Arg Lys
           275                 280                 285
   Ser Ala Ala Gly Tyr Asp Leu Thr Arg Leu Ile Ile Gly Ser Glu Gly
       290                 295                 300
   Thr Leu Gly Val Ile Thr Glu Ile Thr Leu Arg Leu Gln Lys Ile Pro
   305                 310                 315                 320
   Gln His Ser Val Val Ala Val Cys Asn Phe Pro Thr Val Lys Asp Ala
                   325                 330                 335
   Ala Asp Val Ala Ile Ala Thr Met Met Ser Gly Ile Gln Val Ser Arg
               340                 345                 350
   Val Glu Leu Leu Asp Glu Val Gln Ile Arg Ala Ile Asn Met Ala Asn
           355                 360                 365
   Gly Lys Asn Leu Thr Glu Ala Pro Thr Leu Met Phe Glu Phe Ile Gly
       370                 375                 380
   Thr Glu Ala Tyr Thr Arg Glu Gln Thr Gln Ile Val Gln Gln Ile Ala
   385                 390                 395                 400
   Ser Lys His Asn Gly Ser Asp Phe Met Phe Ala Glu Glu Pro Glu Ala
                   405                 410                 415
   Lys Lys Glu Leu Trp Lys Ile Arg Lys Glu Ala Leu Trp Ala Cys Tyr
               420                 425                 430
   Ala Met Ala Pro Gly His Glu Ala Met Ile Thr Asp Val Cys Val Pro
           435                 440                 445
   Leu Ser His Leu Ala Glu Leu Ile Ser Arg Ser Lys Lys Glu Leu Asp
       450                 455                 460
   Ala Ser Ser Leu Leu Cys Thr Val Ile Ala His Ala Gly Asp Gly Asn
   465                 470                 475                 480
   Phe His Thr Cys Ile Met Phe Asp Pro Ser Ser Glu Glu Gln Arg Arg
                   485                 490                 495
   Glu Ala Glu Arg Leu Asn His Phe Met Val His Ser Ala Leu Ser Met
               500                 505                 510
   Asp Gly Thr Cys Thr Gly Glu His Gly Val Gly Thr Gly Lys Met Lys
           515                 520                 525
   Tyr Leu Glu Lys Glu Leu Gly Ile Glu Ala Leu Gln Thr Met Lys Arg
       530                 535                 540
   Ile Lys Lys Thr Leu Asp Pro Asn Asp Ile Met Asn Pro Gly Lys Leu
   545                 550                 555                 560
   Ile Pro Pro His Val Cys Phe
                   565
<210> 9
   <211> 30
   <212> DNA
   <213> artificial
<220>
   <223> primer GO fow
<400> 9
   tacaattgga gatcactaac gttaccgagt 30
<210> 10
   <211> 33
   <212> DNA
   <213> artificial
<220>
   <223> primer GO rev1
<400> 10
   tgggacactc cacgtcctta gtctagacta gta 33
<210> 11
   <211> 34
   <212> DNA
   <213> artificial
<220>
   <223> primer MS fow
<400> 11
   acaccggtcg ctgggaatgt attctgaatc ggca 34
<210> 12
   <211> 36
   <212> DNA
   <213> artificial
<220>
   <223> primer MS rev1
<400> 12
   cacataggca tacatcatcc caggtgagtc gacgtt 36
<210> 13
   <211> 31
   <212> DNA
   <213> artificial
<220>
   <223> primer KatE fow1
<400> 13
   acaccggtcg caacataacg aaaagaaccc a 31
<210> 14
   <211> 31
   <212> DNA
   <213> artificial
<220>
   <223> primer KatE rev1
<400> 14
   acgtcgactc aggcaggaat tttgtcaatc t 31
<210> 15
   <211> 32
   <212> DNA
   <213> artificial
<220>
   <223> primer PGM fow
<400> 15
   taggtaccca atcaacaatg acgtcgacct ac 32
<210> 16
   <211> 32
   <212> DNA
   <213> artificial
<220>
   <223> primer PGM rev
<400> 16
   gagattaaat cgttgccgac gaagcaattg ta 32
<210> 17
   <211> 30
   <212> DNA
   <213> artificial
<220>
   <223> primer rbcS3C fow
<400> 17
   acgagctcat ccagaattgg cgttggatta 30
<210> 18
   <211> 33
   <212> DNA
   <213> artificial
<220>
   <223> primer rbcS3C rev
<400> 18
   agcaacggtg gaagagtcag ttgcaaccgg tat 33

## Description

The present invention is concerned with the improvement of agricobiological traits in plants. More specifically, it relates to a plant cell comprising in its chloroplasts enzymatic activities for converting glycolate into malate, wherein said plant cell comprises a first polypeptide having glycolate oxidase activity, a second polypeptide having malate synthase activity and a third polypeptide having catalase activity. Also encompassed is a plant comprising said plant cells as well as seeds obtainable from the said plants. The present invention, further more, relates to a method for producing a transgenic plant or a plant cell having an increased water use efficiency or increased yield. The present invention contemplates polynucleotides comprising a combination of nucleic acids encoding the aforementioned polypeptides as well as vectors comprising the polynucleotides and uses thereof.

Photosynthetic CO₂ assimilation in C₃-plants is limited by environmental variables including temperature, CO₂ and water availability. Much of this limitation can be attributed to the catalytic properties of ribulose 1,5-bisphosphate carboxylase/oxygenase (RubisCO). Both atmospheric O₂ and O₂ produced by photosystem II compete with CO₂ for binding to the active site of RubisCO. The oxygenation of ribulose 1,5-bisphosphate (RuBP) yields only one molecule of glycerate 3-P, and the remaining two carbons form glycolate 2-P. This glycolate 2-P is the initial substrate of the C2-oxydative photosynthetic cycle (the photorespiratory cycle) leading to the loss of CO₂ [1].

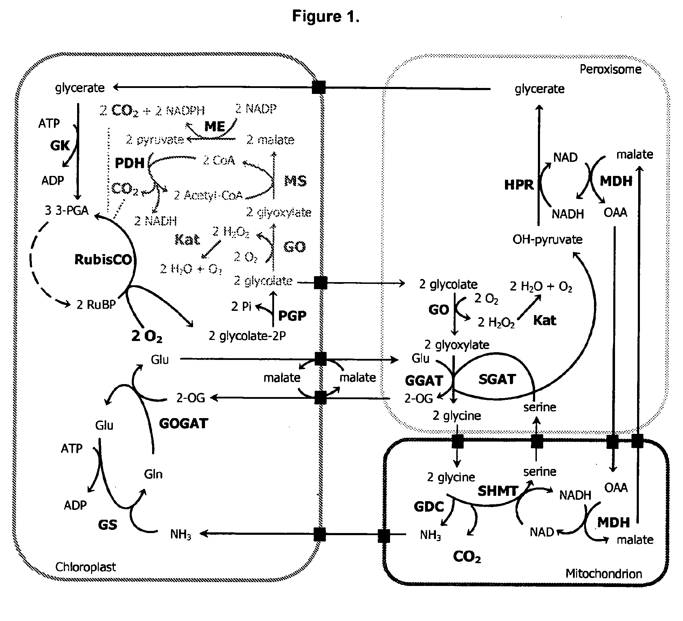

The prime function of the photorespiratory cycle is to salvage glycolate 2-P (Figure 1). In the course of this pathway, two molecules of glycolate 2-P are metabolised to form one molecule of each glycerate 3-P and CO₂, and these carbon compounds are used immediately for the regeneration of RuBP via the Calvin-Benson cycle (the C3-reductive photosynthetic cycle) without the net synthesis of triose phosphates [2, 3].

Glycolate 2-P is metabolized in three distinct cellular compartments namely chloroplasts, peroxisomes, and mitochondria (the site of the release of CO₂ and NH₄⁺), involving numerous enzymatic reactions and transport processes (Figure 1). The photorespiratory pathway relies on the close integration of carbon and nitrogen metabolism in the leaf, because NH₄⁺ is released at the same rate as CO₂ [4]. Since nitrogen is a more valuable resource than carbon, the re-assimilation of photorespired NH₄⁺ is essential for maintaining the nitrogen status in C₃-plants [5]. Ammonia released in the matrix of mitochondria during the course of glycine oxidation is used in the chloroplast by the glutamine synthetase (GS) catalysing the ATP-dependent conversion of glutamate to glutamine. Ferredoxin-dependent glutamate synthase (GOGAT), exclusively located to the chloroplasts of mesophyll cells, catalyses the conversion of glutamine and 2-oxoglutarate to two molecules of glutamate (Figure 1).

One approach to improve the carboxylation activity of RubisCO would be to alter the kinetic constants of RubisCO for CO₂ and O₂. However, the genetic engineering of RubisCO by site-directed mutagenesis to decrease its oxygenase activity or its replacement with more efficient forms, like those from rhodophyte algae in which the CO₂/O₂specificity factor is higher than that of the higher plant RubisCO, was so far only of limited success [6]. Another approach to improve C3 carbon fixation would be to reduce photorespiration directly by manipulation of enzymes in this pathway. From the analysis of insertional mutants it was demonstrated that blocking the photorespiratory metabolism downstream of RubisCO oxygenation is lethal [7-13]. These results suggest that photosynthesis is unlikely to be improved by direct manipulation of levels of enzymes in the photorespiratory pathway. On the other hand, several modes to increase the carboxylase activity of RubisCO by enhancing the actual concentration of CO₂ at its site of action have evolved. Examples are the CO₂ concentrating mechanisms of cyanobacteria and algae [14-15] or the photosynthetic cycle of C₄-plants [16]. The latter provides a CO2 pump, which leads to increased CO₂/O₂ ratio at the site of RubisCO and thus results in a decreased oxygenase activity relative to the carboxylase activity. In C3-plants, RubisCO operates *in vivo* at about 25% of its Vmax while it is believed to operate at or close to its Vmax in C4-plants [16]. Moreover, the specific activities of RubisCO are higher in C4-plants as compared to C₃-plants; hence less RubisCO protein is required to achieve high rates of photosynthesis in C₄-plants. Consequently and due to the fact that RubisCO is a N-expensive enzyme (20-30% of total leaf N in C₃-plants, but only 6% in C₄-plants), C₄-plants display greater photosynthetic nitrogen use efficiency than C₃-plants [17]. Attempts were performed to transfer the advantages of C₄-photosynthesis like high photosynthetic capacity, rapid growth, and increased nitrogen- and water-use efficiencies by overexpression of C4-cycle enzymes in C₃-plants such as *A*. *thaliana,* potato, tobacco and rice [18-21]. Häusler et al. [21] have shown that the overexpression of C4-cycle genes in C₃-plants (potato and tobacco) leads to an attenuation of photorespiration but also to changes in the pattern of endogenous enzymes and contents of UV-protectants. Recently, an attenuation of the flux through photorespiration by completing the pathway with both glyoxylate carboligase and tartronate semialdehyde reductase from *E*. *coli* was shown [22]. The photorespiratory cycle is not only a wasteful process inevitably resulting from the kinetic properties of RubisCO. It may also be regarded as a protecting mechanism for C₃-plants exposed to high temperatures and drought. To mention are the generation of metabolites, such as glycine, serine or one-carbon units, which can be exported from the leaf or used in other metabolic pathways, e.g. glycine for the synthesis of glutathione [23,24] and the prevention of excessive over-reduction of the electron transport chain by dissipating light energy, in particular when C₃-plants are exposed to high light intensities or drought stress (photoprotection; [25, 26]).

WO 03/100066 discloses a plant cell comprising in its chloroplasts enzymatic activities for converting glycolate into glyderate. The plant cell showed decreased photorespiration, increased CO2 assimilation, increased biomass and increased water use efficiency. The document does not disclose the conversion of glycolate to malate.

There is a need for improving CO₂ assimilation in plants and, in particular in C₃-plants in order to improve agricobiological traits of crop plants. However, at the same time the aforementioned drawbacks shall be avoided.

Accordingly, the technical problem underlying the present invention could seen as the provision of means and methods for complying with the aforementioned needs. The technical problem is solved by the subject matter of claims 1 to 29.

Thus, the present invention relates to a plant cell comprising in its chloroplasts enzymatic activities for converting glycolate into malate, wherein said plant cell comprises in its chloroplasts a first polypeptide having glycolat oxidase activity, a second polypeptide having malate synthase activity and a third polypeptide having catalase activity.

The term "plant cell" as used herein encompasses cells from all types of plant tissue, i.e. from leaves, roots, stems, vascular tissue, stalks, calli, cotelydons, stalks, anthers, petioles, seeds or embryonic plant tissue. The host cell according to the present invention can be obtained from any monocot or dicot plant. Preferably, it can be obtained from a model plant, preferably *Arabidopsis thaliana,* or a crop plant selected from the group consisting of oilseed rape, evening primrose, hemp, thistle, peanut, canola, linseed, soybean, safflower, sunflower, borage, maize, wheat, rye, oats, rice, barley, cotton, cassava, pepper, solanaceae plants, preferably, potato, tobacco, eggplant or tomato, vicia species, pea, alfalfa, bushy plants (coffee, cacao, tea), salix species, trees (oil palm, coconut) and perennial grasses and fodder crops. Suitable methods for obtaining host cells from the aforementioned plants as well as conditions for culturing these cells are well known in the art. Preferably, the plant cell is a cell of a C₃-plant.

The term "enzymatic activities" refers, preferably, to enzymatic activities which are introduced into the chloroplasts by introducing one or more polypeptides conferring the said activities, i.e. being capable of converting glycolate into malate, preferably, via glyoxylate as an intermediate. The polypeptides referred to herein shall, therefore, be enzymes exhibiting the aforementioned enzymatic activities when present in a chloroplast. Preferred polypeptides conferring the enzymatic activities necessary for the conversion are disclosed elsewhere in this specification in detail. It is to be understood that the polypeptides referred to herein may also exhibit further biological activities. In order to safeguard the localization of the polypeptides conferring the aforementioned activities into the chloroplast, the polypeptide, preferably, comprises a chloroplast transit peptide. Suitable chloroplast transit peptides are known in the art and are described in Bruce 2000, Trends in Cell Biology 10, 440-447 or Kleffmann 2004, Curr. Biol. 14, 354-362.

The plant cell of the present invention comprises a chloroplast having an endogenous ribulose 1,5-bisphosphate carboxylase/oxygenase activity (RubisCO). Due to the enzymatic properties of RubisCO and their dependencies on the CO₂/O₂ partial pressures, only a limited CO₂ fixation is possible in an unmodified chloroplast. The chloroplast comprised by the plant cell of the present invention is, however, engineered as to allow for conversion of glycolate into malate in the chloroplast by introducing into the chloroplast enzymatic activities which are capable of converting glycolate into glyoxlyate and, subsequently, convert the glyoxylate into malate. By introducing these enzymatic activities into the chloroplast, a complete gylcolate catabolic cycle is established. As a result of this cycle, one molecule of glycolate is converted into two molecules of CO₂ and reducing power in the form of NADPH and NADH (glycolate + O₂ + NAD + NADP → 2 CO₂ + NADH + NADPH + 2H⁺ + H₂O). In this way, the CO₂ is directly released within the chloroplast, thereby increasing the CO₂ partial pressure. This, in turn, reduces the oxygenase activity of RubisCO whereby an autoregulatory cycle is created. To this end, the plant cell, preferably, further comprises an NADP-malic enzyme and a pyruvate dehydrogenase. Preferably, the plant cell of the present invention, thus, has an increased efficiency of CO₂ assimilation compared to a plant cell lacking enzymatic activities for converting glycolate into malate.

Advantageously, as a result of the surprisingly increased CO₂ assimilation, the plant cell of the present invention allows for the generation of plants and, in particular, crop plants, having increased yield and an improved water use efficiency as will be disclosed in more detail elsewhere in this specification. By introducing the aforementioned enzymatic activities and, thus, a complete glycolate catabolic cycle into chloroplasts, CO₂ will be directly released and re-fixed within the chloroplast and thereby reduce the oxygenase activity of RubisCO by increasing the CO₂/O₂ ratio (Figure 1).

In a preferred embodiment of the plant cell of the present invention, the chloroplasts of the plant cell comprises a first polypeptide having glycolate oxidase activity, a second polypeptide having malate synthase activity and a third polypeptide having catalase activity.

A glycolate oxidase as referred to herein shall be capable of converting glycolate into glyoxylate and water peroxide (E.C. 1.1.3.15). A malate synthase shall be capable of synthesising malate by introducing two C-units from Acetyl-CoA into glyoxylate (E.C. 4.1.3.2). A catalase refers to an enzyme being capable of converting water peroxide into water and oxygen (E.C. 1.11.1.6). Any enzyme capable of carrying out these conversions and syntheses can be introduced into the chloroplast according to the present invention. The aforementioned enzymatic activities can be determined by assays well known in the art and described, preferably, in the accompanying Examples below.

Preferably, said first polypeptide is, however, encoded by a first polynucleotide comprising a nucleic acid selected from the group consisting of:
a. a nucleic acid having a nucleotide sequence as shown in SEQ ID No: 1;
b. a nucleic acid encoding a polypeptide having an amino acid sequence as shown in SEQ ID No: 2;
c. a nucleic acid having a nucleotide sequence being at least 50% identical to the nucleotide sequence shown in SEQ ID No: 1 wherein said nucleic acid encodes a polypeptide having glycolate oxidase activity; and
d. a nucleic acid encoding a polypeptide having an amino acid sequence being at least 50% identical to the amino acid sequence shown in SEQ ID No:2, wherein said polypeptide has glycolate oxidase activity.

Said second polypeptide is, preferably, encoded by a second polynucleotide comprising a nucleic acid selected from the group consisting of:
a. a nucleic acid having a nucleotide sequence as shown in SEQ ID No: 3;
b. a nucleic acid encoding a polypeptide having an amino acid sequence as shown in SEQ ID No: 4;
c. a nucleic acid having a nucleotide sequence being at least 40% identical to the nucleotide sequence shown in SEQ ID No: 3 wherein said nucleic acid encodes a polypeptide having malate synthase activity; and
d. a nucleic acid encoding a polypeptide having an amino acid sequence being at least 40% identical to the amino acid sequence shown in SEQ ID No: 4, wherein said polypeptide has malate synthase activity.

The third polypeptide, preferably, is encoded by a third polynucleotide comprising a nucleic acid selected from the group consisting of:
a. a nucleic acid having a nucleotide sequence as shown in SEQ ID No: 5;
b. a nucleic acid encoding a polypeptide having an amino acid sequence as shown in SEQ ID No: 6;
c. a nucleic acid having a nucleotide sequence being at least 50% identical to the nucleotide sequence shown in SEQ ID No: 5 wherein said nucleic acid encodes a polypeptide having catalase activity; and
d. a nucleic acid encoding a polypeptide having an amino acid sequence being at least 50% identical to the amino acid sequence shown in SEQ ID No: 6, wherein said polypeptide has catalase activity.

The term "polynucleotide" as used herein refers to a linear or circular nucleic acid molecule. It encompasses DNA as well as RNA molecules. The polynucleotide of the present invention shall be provided, preferably, either as an isolated polynucleotide (i.e. isolated from its natural context) or in genetically modified form. The term encompasses single as well as double stranded polynucleotides. Moreover, comprised are also chemically modified polynucleotides including naturally occurring modified polynucleotides such as glycosylated or methylated polynucleotides or artificial modified one such as biotinylated polynucleotides. The polynucleotide of the present invention is characterized in that it shall encode a polypeptide as referred to above. The polynucleotide, preferably, has a specific nucleotide sequence as mentioned above. Moreover, due to the degeneracy of the genetic code, polynucleotides are encompassed which encode a specific amino acid sequence as recited above.

Moreover, the term "polynucleotide" as used in accordance with the present invention further encompasses variants of the aforementioned specific polynucleotides. Said variants may represent orthologs, paralogs or other homologs of the polynucleotide of the present invention. The polynucleotide variants, preferably, comprise a nucleic acid sequence characterized in that the sequence can be derived from the aforementioned specific nucleic acid sequences by at least one nucleotide substitution, addition and/or deletion whereby the variant nucleic acid sequence shall still encode a polypeptide having the activity as specified above. Variants also encompass polynucleotides comprising a nucleic acid sequence which is capable of hybridizing to the aforementioned specific nucleic acid sequences, preferably, under stringent hybridization conditions. These stringent conditions are known to the skilled worker and can be found in Current Protocols in Molecular Biology, John Wiley & Sons, N. Y. (1989), 6.3.1-6.3.6. A preferred example for stringent hybridization conditions are hybridization conditions in 6 x sodium chloride/sodium citrate (= SSC) at approximately 45°C, followed by one or more wash steps in 0.2 x SSC, 0.1% SDS at 50 to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of 0.1 to 5 x SSC (pH 7.2). If organic solvent is present in the abovementioned buffer, for example 50% formamide, the temperature under standard conditions is approximately 42°C. The hybridization conditions for DNA:DNA hybrids are preferably for example 0.1 x SSC and 20°C to 45°C, preferably between 30°C and 45°C. The hybridization conditions for DNA:RNA hybrids are preferably, for example, 0.1 x SSC and 30°C to 55°C, preferably between 45°C and 55°C. The abovementioned hybridization temperatures are determined for example for a nucleic acid with approximately 100 bp (= base pairs) in length and a G + C content of 50% in the absence of formamide. The skilled worker knows how to determine the hybridization conditions required by referring to textbooks such as the textbook mentioned above, or the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical Approach", IRL Press at Oxford University Press, Oxford. Alternatively, polynucleotide variants are obtainable by PCR-based techniques such as mixed oligonucleotide primer- based amplification of DNA, i.e. using degenerated primers against conserved domains of the polypeptides of the present invention. Conserved domains of the polypeptide of the present invention may be identified by a sequence comparison of the nucleic acid sequence of the polynucleotide or the amino acid sequence of the polypeptide of the present invention with sequences of other members of the enzyme families referred to in accordance with this invention. Oligonucleotides suitable as PCR primers as well as suitable PCR conditions are described in the accompanying Examples. As a template, DNA or cDNA from bacteria, fungi, plants or animals may be used. Further, variants include polynucleotides comprising nucleic acid sequences which are at least 70%, at least 75%, at least 80%, at least 85%, at least 90%, at least 95%, at least 98% or at least 99% identical to the specific nucleic acid sequences. Moreover, also encompassed are polynucleotides which comprise nucleic acid sequences encoding amino acid sequences which are at least 70%, at least 75%, at least 80%, at least 85%, at least 90%, at least 95%, at least 98% or at least 99% identical to the specific amino acid sequences referred to herein. The percent identity values are, preferably, calculated over the entire amino acid or nucleic acid sequence region. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp ( Higgins 1989, CABIOS, 5 1989: 151-153) or the programs Gap and BestFit (Needleman 1970, J. Mol. Biol. 48; 443-453 and Smith 198, Adv. Appl. Math. 2; 482-489), which are part of the GCG software packet from Genetics Computer Group, 575 Science Drive, Madison, Wisconsin, USA 53711, version 1991, are to be used. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

A polynucleotide comprising a fragment of any of the aforementioned nucleic acid sequences is.also encompassed as a polynucleotide of the present invention. The fragment shall encode a polypeptide which still has the activity as specified above. Accordingly, the polypeptide may comprise or consist of the domains of the polypeptide of the present invention conferring the said biological activity. A fragment as meant herein, preferably, comprises at least 50, at least 100, at least 250 or at least 500 consecutive nucleotides of any one of the aforementioned nucleic acid sequences or encodes an amino acid sequence comprising at least 20, at least 30, at least 50, at least 80, at least 100 or at least 150 consecutive amino acids of any one of the aforementioned amino acid sequences.

The polynucleotides of the present invention either essentially consist of the aforementioned nucleic acid sequences or comprise the aforementioned nucleic acid sequences. Thus, they may contain further nucleic acid sequences as well. Specifically, the polynucleotides of the present invention may encode fusion proteins wherein one partner of the fusion protein is a polypeptide being encoded by a nucleic acid sequence recited above. Such fusion proteins may comprise as additional part peptide sequences for monitoring expression (e.g., green, yellow, blue or red fluorescent proteins, alkaline phosphatase and the like) or so called "tags" which may serve as a detectable marker or as an auxiliary measure for purification purposes. Tags for the different purposes are well known in the art and comprise FLAG-tags, 6-histidine-tags, MYC-tags and the like.

More preferably, the first, second and/or third polypeptide(s) referred to above are expressed from heterologous polynucleotides, i.e. from polynucleotides which have been either transiently, e.g., by using an expression vector, or stably, e.g., by using T- or P-DNA insertion, introduced into the plant cell. The term "heterologous" as used herein means that the polynucleotides do not occur naturally in the plant cell. The term, thus, encompasses modified or unmodified polynucleotides which are derived from different organisms or modified polynucleotides derived from the plant cell of the invention. It is to be understood that the heterologous polynucleotide shall either comprise expression control sequences which allow for expression in the plant cell or sequences which allow for integration of the heterologous polynucleotide at a locus in the genome of the plant cell where the expression of the heterologous polynucleotide will be governed by endogenous expression control sequences of the plant cell. Preferably, the heterologous polynucleotide comprises a nucleic acid having a nucleic acid sequence of the first, the second or the third polynucleotide as referred to before, i.e. a polynucleotide encoding a glyoxylate oxidase, a polynucleotide encoding malate synthase or a polynucleotide encoding a catalase, two of the said nucleic acids or all three of them. By introducing the aforementioned heterologous polynucleotides, transgenic plant cells are generated. Such transgenic plant cells may be obtained by transformation techniques as published, and cited, in: Plant Molecular Biology and Biotechnology (CRC Press, Boca Raton, Florida), chapter 6/7, pp.71-119 (1993); F.F. White, Vectors for Gene Transfer in Higher Plants; in: Transgenic Plants, vol. 1, Engineering and Utilization, Ed.: Kung and R. Wu, Academic Press, 1993, 15-38; B. Jenes et al., Techniques for Gene Transfer, in: Transgenic Plants, vol. 1, Engineering and Utilization, Ed.: Kung and R. Wu, Academic Press (1993), 128-143; Potrykus, Annu. Rev. Plant Physiol. Plant Molec. Biol. 42 (1991), 205-225. Preferably, transgenic plants can be obtained by T-DNA-mediated or P-DNA-mediated transformation. Such vector systems are, as a rule, characterized in that they contain at least the vir genes, which are required for the Agrobacterium-mediated transformation, and the sequences which delimit the T- or P-DNA (T-DNA border or P-DNA border). Suitable vectors are described elsewhere in the specification in detail.

The present invention also relates to a plant comprising the plant cell of the present invention.

The plant of the present invention, preferably, develops from the plant cell of the present invention. Thus, it is envisaged that all plant cells of the plant are plant cells according to the present invention, i.e., comprise in their chloroplasts enzymatic activities for converting glycolate into malate. As set forth above for the plant cells of the invention, preferred plants of the present invention are selected from the group consisting of: model plants, preferably *Arabidopsis thaliana,* or crop plants, preferably, oillseed rape, evening primrose, hemp, thistle, peanut, canola, linseed, soybean, safflower, sunflower, borage, maize, wheat, rye, oats, rice, barley, cotton, cassava, pepper, solanaceae plants, preferably, potato, tobacco, eggplant or tomato, vicia species, pea, alfalfa, bushy plants (coffee, cacao, tea), salix species, trees (oil palm, coconut) and perennial grasses and fodder crops. Preferably, the plant of the present invention is a C₃ plant.

Preferably, the plant of the present invention has increased CO₂ assimilation compared to a plant lacking a plant cell of the present invention. CO₂ assimilation as used herein refers to the conversion of CO₂ and water into organic molecules during photosynthesis. The chemical reactions in this context are well known in the art and are described in standard biochemical text books. An increase in CO₂ assimilation can be determined by techniques well known in the art and described in, preferably, in the accompanying Examples below. Preferably, the increase is statistically significant. Whether an increase is statistically significant can be determined by well known statistical tests including, e.g., Student's t-test, Mann-Whitney test etc.. More preferably, said increase is an increase of at least 10%, at least 20%, at least 30%, at least 40% or at least 50% in assimilated CO₂. A plant lacking a plant cell of the present invention as meant herein, preferably, refers to an unmodified control plant of the same variety as the plant of the present invention.

Also preferably, the plant of the present invention has an attenuated photorespiration compared to a plant lacking a plant cell of the present invention. Attenuation of the activity of the photorespiratory pathway can be determined by well known techniques. Suitable assays are disclosed in preferably, in the accompanying Examples below. Attenuation as referred to herein is, preferably, a statistically significant attenuation.

The plant of the present invention, preferably, has increased water use efficiency compared to a plant lacking a plant cell of the present invention. The water use efficiency is increased since the stomata in the leaves can be kept closer as a result of the increased CO₂ assimilation. Accordingly, the loss of water by evaporation is reduced. An increase in water use efficiency can be determined by techniques well known in the art and described in, preferably, in the accompanying Examples below. Preferably, the increase is statistically significant.

More preferably, said increase is an increase of at least 10%, at least 20%, at least 30%, at least 40% or at least 50%.

Preferably, the plant of the present invention produces an increased number of leaves compared to a plant lacking a plant cell of the present invention. The number of leaves may be either counted or determined based on the fresh or dry weight of the leaves. Preferably, the increase in the number of leaves is statistically significant. More preferably, said increase is an increase in the numbers of leaves of at least 10%, at least 20%, at least 30%, at least 40% or at least 50%.

Furthermore, the plant of the present invention has an increased yield compared to a plant lacking a plant cell of the present invention. The term "yield" as used herein encompasses an increase in biomass (fresh or dry weight) of a plant part or the entire plant, and particularly, harvestable parts of the plant. The increase in biomass may be aboveground or underground. An increase in biomass underground may be due to an increase in the biomass of plant parts, such as tubers, rhizomes, bulbs etc. Particularly preferred is an increase in any one or more of the following: increased root biomass, increased root volume, increased root number, increased root diameter and increased root length. The term increased yield also encompasses an increase in seed yield. An increase in seed yield includes: (i) increased total seed yield, which includes an increase in seed biomass (seed weight) and which may be an increase in the seed weight per plant or on an individual seed basis; (ii) increased number of flowers ("florets") per panicle; (iii) increased number of filled seeds; (iv) increased seed size; (v) increased seed volume; (vi) increased individual seed area; (vii) increased individual seed length and/or width; (viii) increased harvest index, which is expressed as a ratio of the yield of harvestable parts, such as seeds, over the total biomass; (ix) increased fill rate, (which is the number of filled seeds divided by the total number of seeds and multiplied by 100); and (x) increased thousand kernel weight (TKW), which is extrapolated from the number of filled seeds counted and their total weight. An increased TKW may result from an increased seed size and/or seed weight. An increased TKW may result from an increase in embryo size and/or endosperm size. Preferably, the increase in yield is statistically significant. More preferably, said increase is an increase of at least 10%, at least 20%, at least 30%, at least 40% or at least 50% in yield.

Finally, because this engineered pathway is localized to the chloroplasts, CO₂ can directly be re-fixed and less ammonia is released. Thus, a lesser extent of re-assimilation of ammonia is required and an improvement in the nitrate use efficiency also will occur in the plant of the present invention.

The present invention encompasses, furthermore, a seed obtainable from a plant of the present invention. The seed of the present invention is capable of generating (i.e. developing into) a plant of the present invention and, specifically, a plant having the traits as defined above.

Plants or plant cells of the present invention are, preferably, transgenic plants or plant cells. Accordingly, the present invention contemplates a method for producing a transgenic plant or a plant cell having an increased water use efficiency compared to a corresponding non-transgenic plant or plant cell, said method comprises introducing in the chloroplasts of the transgenic plant or plant cell a first polypeptide having glycolate oxidase activity, a second polypeptide having malate synthase activity and a third polypeptide having catalase activity as defined above.

The introduction of the polypeptides is, preferably, achieved by introducing heterologous polynucleotides encoding the aforementioned polypeptides as discussed elsewhere in this specification in more detail. This includes transient introduction in expression vectors or stable integration into the genome of the plant cells via, e.g., T- or P-DNA insertion. It is to be understood that one heterologous polynucleotide comprising nucleic acids encoding the all of the aforementioned polypeptides may be introduced. Alternatively, separate heterologous polynucleotides each comprising a nucleic acid encoding a polypeptide of the aforementioned polypeptides may be introduced. Thus, the method, preferably, comprises the steps of:
a. introducing at least one heterologous polynucleotide encoding the said polypeptides into the plant of plant cell; and
b. expressing said polypeptides from the said at least one polynucleotide.

The present invention also contemplates a method for producing a transgenic plant or plant cell having increased yield compared to a corresponding non-transgenic plant or plant cell, said method comprises introducing in the chloroplasts of the transgenic plant or plant cell a first polypeptide having glycolate oxidase activity, a second polypeptide having malate synthase activity and a third polypeptide having catalase activity as defined above. The method, preferably, also comprises the steps of:
a. introducing at least one heterologous polynucleotide encoding the said polypeptides into the plant of plant cell; and
b. expressing said polypeptides from the said at laest one polynucleotide.

The present invention encompasses a polynucleotide comprising in combination a nucleic acid selected from the group consisting of:
a. a nucleic acid having a nucleotide sequence as shown in SEQ ID No: 1;
b. a nucleic acid encoding a polypeptide having an amino acid sequence as shown in SEQ ID No: 2;
c. a nucleic acid having a nucleotide sequence being at least 50% identical to the nucleotide sequence shown in SEQ ID No: 1 wherein said nucleic acid encodes a polypeptide having glycolate oxidase activity; and
d. a nucleic acid encoding a polypeptide having an amino acid sequence being at least 50% identical to the amino acid sequence shown in SEQ ID No:2, wherein said polypeptide has glycolate oxidase activity;
a nucleic acid selected from the group consisting of:
a. a nucleic acid having a nucleotide sequence as shown in SEQ ID No: 3;
b. a nucleic acid encoding a polypeptide having an amino acid sequence as shown in SEQ ID No: 4;
c. a nucleic acid having a nucleotide sequence being at least 40% identical to the nucleotide sequence shown in SEQ ID No: 3 wherein said nucleic acid encodes a polypeptide having malate synthase activity; and
d. a nucleic acid encoding a polypeptide having an amino acid sequence being at least 40% identical to the amino acid sequence shown in SEQ ID No: 4, wherein said polypeptide has malate synthase activity; and
a nucleic acid selected from the group consisting of:
a. a nucleic acid having a nucleotide sequence as shown in SEQ ID No: 5;
b. a nucleic acid encoding a polypeptide having an amino acid sequence as shown in SEQ ID No: 6;
c. a nucleic acid having a nucleotide sequence being at least 50% identical to the nucleotide sequence shown in SEQ ID No: 5 wherein said nucleic acid encodes a polypeptide having catalase activity; and
d. a nucleic acid encoding a polypeptide having an amino acid sequence being at least 50% identical to the amino acid sequence shown in SEQ ID No: 6, wherein said polypeptide has catalase activity.

By introducing any one of the aforementioned polynucleotides of the present invention as a heterologous polynucleotide into a plant cell or plant, the traits referred to in accordance with the present invention will be conferred to the said plant or plant cell.

The present invention also contemplates a vector comprising one of the aforementioned polynucleotides of the present invention.

The term "vector", preferably, encompasses phage, plasmid, viral or retroviral vectors as well as artificial chromosomes, such as bacterial or yeast artificial chromosomes. Moreover, the term also relates to targeting constructs which allow for random or site- directed integration of the targeting construct into genomic DNA. Such target constructs, preferably, comprise DNA of sufficient length for either homologous or heterologous recombination as described in detail below. The vector encompassing the polynucleotides of the present invention, preferably, further comprises selectable markers for propagation and/or selection in a host. The vector may be incorporated into a host cell by various techniques well known in the art. If introduced into a host cell, the vector may reside in the cytoplasm or may be incorporated into the genome. In the latter case, it is to be understood that the vector may further comprise nucleic acid sequences which allow for homologous recombination or heterologous insertion. Vectors can be introduced into prokaryotic or eukaryotic cells via conventional transformation or transfection techniques. The terms "transformation" and "transfection", conjugation and transduction, as used in the present context, are intended to comprise a multiplicity of prior-art processes for introducing foreign nucleic acid (for example DNA) into a host cell, including calcium phosphate, rubidium chloride or calcium chloride co-precipitation, DEAE-dextran-mediated transfection, lipofection, natural competence, carbon-based clusters, chemically mediated transfer, electroporation or particle bombardment (e.g., "gene-gun"). Suitable methods for the transformation or transfection of host cells, including plant cells, can be found in Sambrook et al. (Molecular Cloning: A Laboratory Manual, 2nd ed., Cold Spring Harbor Laboratory, Cold Spring Harbor Laboratory Press, Cold Spring Harbor, NY, 1989) and other laboratory manuals, such as Methods in Molecular Biology, 1995, Vol. 44, Agrobacterium protocols, Ed.: Gartland and Davey, Humana Press, Totowa, New Jersey. Alternatively, a plasmid vector may be introduced by heat shock or electroporation techniques. Should the vector be a virus, it may be packaged in vitro using an appropriate packaging cell line prior to application to host cells. Retroviral vectors may be replication competent or replication defective. In the latter case, viral propagation generally will occur only in complementing host cells.

Preferably, the vector referred to herein is suitable as a cloning vector, i.e. replicable in microbial systems. Such vectors ensure efficient cloning in bacteria and, preferably, yeasts or fungi and make possible the stable transformation of plants. Those which must be mentioned are, in particular, various binary and co-integrated vector systems which are suitable for the T-DNA-mediated or P-DNA mediated transformation. Such vector systems are, as a rule, characterized in that they contain at least the vir genes, which are required for the Agrobacterium-mediated transformation, and the sequences which delimit the T-DNA or P-DNA (T-DNA or P-DNA borders). These vector systems, preferably, also comprise further cis-regulatory regions such as promoters and terminators and/or selection markers with which suitable transformed host cells or organisms can be identified. While co-integrated vector systems have vir genes and T-DNA sequences arranged on the same vector, binary systems are based on at least two vectors, one of which bears vir genes, but no T-DNA, while a second one bears T-DNA, but no vir gene. As a consequence, the last-mentioned vectors are relatively small, easy to manipulate and can be replicated both in E. coli and in Agrobacterium. These binary vectors include vectors from the pBIB-HYG, pCAMBIA, pPZP, pBecks, pGreen series. Preferably used in accordance with the invention is pGreen II.. An overview of binary vectors and their use can be found in Hellens 2000, Trends in Plant Science 5, 446-451. Furthermore, by using appropriate cloning vectors, the polynucleotide of the invention can be introduced into host cells or organisms such as plants or animals and, thus, be used in the transformation of plants, such as those which are published, and cited, in: Plant Molecular Biology and Biotechnology (CRC Press, Boca Raton, Florida), chapter 6/7, pp. 71-119 (1993); F.F. White, Vectors for Gene Transfer in Higher Plants; in: Transgenic Plants, vol. 1, Engineering and Utilization, Ed.: Kung and R. Wu, Academic Press, 1993, 15-38; B. Jenes et al., Techniques for Gene Transfer, in: Transgenic Plants, vol. 1, Engineering and Utilization, Ed.: Kung and R. Wu, Academic Press (1993), 128-143; Potrykus, Annu. Rev. Plant Physiol. Plant Molec. Biol. 42 (1991), 205-225.

More preferably, the vector of the present invention is an expression vector. In such an expression vector, the polynucleotide comprises an expression cassette as specified above allowing for expression in eukaryotic cells or isolated fractions thereof. An expression vector may, in addition to the polynucleotide of the invention, also comprise further regulatory elements including transcriptional as well as translational enhancers. Preferably, the expression vector is also a gene transfer or targeting vector. Expression vectors derived from viruses such as retroviruses, vaccinia virus, adeno-associated virus, herpes viruses, or bovine papilloma virus, may be used for delivery of the polynucleotides or vector of the invention into targeted cell population. Methods which are well known to those skilled in the art can be used to construct recombinant viral vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994).

Suitable expression vector backbones are, preferably, derived from expression vectors known in the art such as Okayama-Berg cDNA expression vector pcDV1 (Pharmacia), pCDM8, pRc/CMV, pcDNA1, pcDNA3 (Invitrogene) or pSPORT1 (GIBCO BRL). Further examples of typical fusion expression vectors are pGEX (Pharmacia Biotech Inc; Smith, D.B., and Johnson, K.S. (1988) Gene 67:31-40), pMAL (New England Biolabs, Beverly, MA) and pRIT5 (Pharmacia, Piscataway, NJ), where glutathione S-transferase (GST), maltose E-binding protein and protein A, respectively, are fused with the nucleic acid of interest encoding a protein to be expressed. The target gene expression of the pTrc vector is based on the transcription from a hybrid trp-lac fusion promoter by host RNA polymerase. The target gene expression from the pET 11 d vector is based on the transcription of a T7-gn10-lac fusion promoter, which is mediated by a coexpressed viral RNA polymerase (T7 gn1). This viral polymerase is provided by the host strains BL21 (DE3) or HMS174 (DE3) from a resident λ-prophage which harbors a T7 gn1 gene under the transcriptional control of the lacUV 5 promoter. Examples of vectors for expression in the yeast S. cerevisiae comprise pYeDesaturased (Baldari et al. (1987) Embo J. 6:229-234), pMFa (Kurjan and Herskowitz (1982) Cell 30:933-943), pJRY88 (Schultz et al. (1987) Gene 54:113-123) and pYES2 (Invitrogen Corporation, San Diego, CA). Vectors and processes for the construction of vectors which are suitable for use in other fungi, such as the filamentous fungi, comprise those which are described in detail in: van den Hondel, C.A.M.J.J., & Punt, P.J. (1991) "Gene transfer systems and vector development for filamentous fungi, in: Applied Molecular Genetics of fungi, J.F. Peberdy et al., Ed., pp. 1-28, Cambridge University Press: Cambridge, or in: More Gene Manipulations in Fungi (J.W. Bennett & L.L. Lasure, Ed., pp. 396-428: Academic Press: San Diego). Further suitable yeast vectors are, for example, pAG-1, YEp6, YEp13 or pEMBLYe23. As an alternative, the polynucleotides of the present invention can be also expressed in insect cells using baculovirus expression vectors. Baculovirus vectors which are available for the expression of proteins in cultured insect cells (for example Sf9 cells) comprise the pAc series (Smith et al. (1983) Mol. Cell Biol. 3:2156-2165) and the pVL series (Lucklow and Summers (1989) Virology 170:31-39).

Expression vectors allowing expression in plant cells comprise those which are described in detail in: Becker, D., Kemper, E., Schell, J., and Masterson, R. (1992) "New plant binary vectors with selectable markers located proximal to the left border", Plant Mol. Biol. 20:1195-1197; and Bevan, M.W. (1984) "Binary Agrobacterium vectors for plant transformation", Nucl. Acids Res. 12:8711-8721; Vectors for Gene Transfer in Higher Plants; in: Transgenic Plants, Vol. 1, Engineering and Utilization, Ed.: Kung and R. Wu, Academic Press, 1993, p. 15-38. A plant expression cassette, preferably, comprises regulatory sequences which are capable of controlling the gene expression in plant cells and which are functionally linked so that each sequence can fulfill its function, such as transcriptional termination, for example polyadenylation signals. Preferred polyadenylation signals are those which are derived from Agrobacterium tumefaciens T-DNA, such as the gene 3 of the Ti plasmid pTiACH5, which is known as octopine synthase (Gielen et al., EMBO J. 3 (1984) 835 et seq.) or functional equivalents of these, but all other terminators which are functionally active in plants are also suitable. Since plant gene expression is very often not limited to transcriptional levels, a plant expression cassette preferably comprises other functionally linked sequences such as translation enhancers, for example the overdrive sequence, which comprises the 5'-untranslated tobacco mosaic virus leader sequence, which increases the protein/RNA ratio (Gallie et al., 1987, Nucl. Acids Research 15:8693-8711). Other preferred sequences for the use in functional linkage in plant gene expression cassettes are targeting sequences which are required for targeting the gene product into its relevant cell compartment. In the present case, in particular, the relevant compartment are the chloroplasts.

The present invention also relates to a composition comprising
a first polynucleotide comprising a nucleic acid selected from the group consisting of:
a. a nucleic acid having a nucleotide sequence as shown in SEQ ID No: 1;
b. a nucleic acid encoding a polypeptide having an amino acid sequence as shown in SEQ ID No: 2;
c. a nucleic acid having a nucleotide sequence being at least 50% identical to the nucleotide sequence shown in SEQ ID No: 1 wherein said nucleic acid encodes a polypeptide having glycolate oxidase activity; and
d. a nucleic acid encoding a polypeptide having an amino acid sequence being at least 50% identical to the amino acid sequence shown in SEQ ID No:2, wherein said polypeptide has glycolate oxidase activity;
a second polynucleotide comprising a nucleic acid selected from the group consisting of:
a. a nucleic acid having a nucleotide sequence as shown in SEQ ID No: 3;
b. a nucleic acid encoding a polypeptide having an amino acid sequence as shown in SEQ ID No: 4;
c. a nucleic acid having a nucleotide sequence being at least 40% identical to the nucleotide sequence shown in SEQ ID No: 3 wherein said nucleic acid encodes a polypeptide having malate synthase activity; and
d. a nucleic acid encoding a polypeptide having an amino acid sequence being at least 40% identical to the amino acid sequence shown in SEQ ID No: 4, wherein said polypeptide has malate synthase activity; and
a third polynucleotide comprising a nucleic acid selected from the group consisting of:
a. a nucleic acid having a nucleotide sequence as shown in SEQ ID No: 5;
b. a nucleic acid encoding a polypeptide having an amino acid sequence as shown in SEQ ID No: 6;
c. a nucleic acid having a nucleotide sequence being at least 50% identical to the nucleotide sequence shown in SEQ ID No: 5 wherein said nucleic acid encodes a polypeptide having catalase activity; and
d. a nucleic acid encoding a polypeptide having an amino acid sequence being at least 50% identical to the amino acid sequence shown in SEQ ID No: 6, wherein said polypeptide has catalase activity.

As set forth elsewhere in this specification, such compositions comprises heterologous polynucleotides which can be used to confer the enzymatic activities to a plant or plant cell which are required for improving the traits referred to herein.

It will be understood that the aforementioned polynucleotides, vectors or compositions of the present invention are, preferably, to be used for conferring increased water use efficiency to a plant or plant cell or for conferring increased yield to a plant or plant cell.

### FIGURES

**Figure 1****.** The photorespiratory carbon and nitrogen cycle (black) of a C3-plant shortcircuited by the novel glycolate catabolic pathway (red). The transgenic enzymes introduced into *A. thaliana* chloroplasts are highlighted in green. Kat: catalase; GDC: glycine decarboxylase; GGAT: glutamate-glyoxylate aminotransferase; GK: glycerate kinase; GO: glycolate oxidase; GOGAT: glutamate-oxoglutarate aminotransferase; GS: glutamine synthetase; HPR: hydroxypyruvate reductase; MDH: NAD-malate dehydrogenase; ME: NADP-malic enzyme; MS: malate synthase; PDH: pyruvate dehydrogenase; PGP: phosphoglycolate phosphatase; SGAT: serine-glutamate aminotransferase; SHMT: serine hydroxymethyl transferase. Adapted from [3].
**Figure 2****.** Enzymatic activities in leaf extracts of transgenic and wild-type plants. A) Glycolate oxidase activity. B) Malate synthase activity. C) Catalase activity. Error bars indicate the SE of three different determinations.
**Figure 3****.** Growth characteristics of 6-week-old plants grown in short-day conditions at ambient (380 ppm) and high CO₂ (2,000 ppm) concentrations.
**Figure 4****.** Differences in the accumulation of starch in leaves of 6-week-old transgenic and wild-type plants grown in short-days (A-C); A) at 380 ppm; B) at 2,000 ppm; C) plants transferred for six days at 600 µmol quanta m-2 s-1 during the light period. D) Phenotype of the plants treated as in C.

The following examples merely illustrate the invention. They should, whatsoever, not be construed as limiting the scope of protection.

### EXAMPLES

### Example 1: PCR amplification of the transgenes

The cDNAs corresponding to the *glycolate oxidase 2* from *A. thaliana* leaves (GO, At3g14420) and the *malate synthase* from pumpkin cotyledons (MS, X56948) and the genomic sequence from *E*. *coli* catalase (*KatE*; M55161) were amplified using Platinum Pfx DNA polymerase (Invitrogen, Karlsruhe, Germany) and cloned into pGEMT-Easy (Promega, Mannheim, Germany) or alternatively, pCR-Blunt II-TOPO (Invitrogen). In the cases of GO and MS, the nucleotides encoding for the last amino acids (A/SRL), which presumably represent the peroxisomal targeting signal (31, 32), were omitted. The following primer combinations were used: GO fow (5'- TACAATTGGAGATCACTAACGTTAC-CGAGT-3' SEQ ID NO: 9) and GO rev1 (5'-TGGGACACTCCACGTCCTTAGTCTAGACTAGTA-3' SEQ ID NO: 10), MS fow (5'- ACACCGGTCGCTGGGAATGTATTCTGAATCGGCA-3' SEQ ID NO: 11) and MS rev1 (5'- CACATAGGCATACATCATCCCAGGTGAGTCGACGTT-3' SEQ ID NO: 12) KatE fow1 (5'- ACACCGGTCGCAACATAACGAAAAGAAC-CCA-3'SEQ ID NO: 13) and KatE rev1 (5'- ACGTCGACTCAGGCAG-GAATTTTGTCAATCT-3' SEQ ID NO: 14). To follow the cloning strategy, primers were designated to introduce unique Munl and Spel sites at the 5' and 3' ends of GO and AgeI and SalI sites at the 5' and 3' ends of MS and KatE. In order to target GO to chloroplasts, the stromal targeting presequences from the *A. thaliana phosphoglucomutase* (PGM; 225 bp) was amplified by PCR using the following primers: PGM fow (5'-TAGGTACCCAATCAACAATGACGTCGACCTAC-3' SEQ ID NO: 15) and PGM rev (5'-GAGATTAAATCGTTGCCGACGAAGCAATTGTA-3' SEQ ID NO: 16).

The oligonucleotides were designated to introduce unique KpnI and MunI sites at the 5' and 3'ends. The fragment obtained was cloned upstream the GO cDNA. To target MS and KatE to the chloroplast a fragment containing the *tomato-RubisCO-small subunit* (rbcS3C; X66072) promoter (715 bp) and transit peptide (172 bp) was amplified by PCR using genomic DNA and the following primers: rbcS3C fow (5'- ACGAGCTCATCCAGAATTGGCGTTGGATTA-3' SEQ ID NO: 17) and rbcS3C rev (5'- AGCAACGGTGGAA-GAGTCAGTTGCAACCGGTAT-3' SEQ ID NO: 18). The primers were designated to introduce unique SacI and Agel sites at the 5' and 3' ends. The fragment obtained was inserted upstream the MS and *KatE* coding regions. All plasmid constructions were sequenced using the PRISM fluorescent dye-terminator system (Applied Biosystems, Darmstadt, Germany) to exclude any possible mutation that could be created by the polymerase action.

### Example 2: Construction of binary vectors

To direct the expression in *A. thaliana,* the DNAs encoding the plastidic precursor of the enzymes were cloned into a modified version of the binary vector pGreenlI [33, 34]. The DNAs coding for the enzymes were cloned into the binary vector using different strategies. In the case of *MS* and *KatE,* the CaMV35S promoter was cut out of the vector and the tomato-rbcS3C promoter was used to direct the expression. The selection marker genes nosKan (kanamycin resistance), nosHyg (hygromycin resistance) and nosBAR 18 (BASTA resistance) were cloned into the Stul-site within the basic pGreenII vector. GO was cloned into the pGreenII 35S-nosKan vector, MS into the pGreenII rbcS3C-nosHyg vector and *KatE* into the pGreenII rbcS3C-nosBAR vector, the resulting plasmids were called *35S*:*GO*, *rbcS3C:MS* and *rbcS3C:KatE.*

### Example 3: Transformation of A. thaliana and selection of transformants

The binary vectors *35S:GO, rbcS3C:MS* and *rbcS3C:KatE* were electroporated into *Agrobacterium tumefaciens* GV3101 bearing the helper plasmid pSoup and used to transform *A*. *thaliana* plants (Colombia ecotype, Col-0) via vacuum infiltration [35]. Transformed seeds were selected either by resistance to kanamycin, hygromycin or BASTA. Leaf material was collected from selected plants and DNA was extracted for PCR analyses. Plants containing the transgenes were allowed to self-pollinate. Transgenic lines were subjected to two more rounds of selection and characterization by means of PCR and enzymatic activity assays.

Plants were grown, in principle, under a 8h-light/16h-dark regime at photosynthetically active photon flux densities (PPFD) of 100 or 600 µmol quanta m-2 s-1 (normal and elevated light intensities, respectively) and adjusted to 22°C day/18°C night temperatures. For the growth at non-photorespiratory conditions, three-week-old plants grown in normal conditions were transferred to a chamber with a CO₂ concentration of 2,000 ± 200 ppm.

### Example 4: Production of GO-MS and GMK transformants

GO-MS lines were produce by transformation of GO (line 5, GO5) with the vector *rbcS3C:MS*. The lines containing all three genes, GMK lines, were produced by retransformation of GO-MS (line 6, GO-MS6) with the vector *rbcS3C:KatE.* In all cases, the lines were selected taking advantage of the different resistances of the transgenes and corroborated by PCR, Southern blot and determination of enzymatic activities.

### Example 5: Chloroplast isolation, preparation of extracts and enzymatic measurements

Intact chloroplasts were isolated as described by Kebeish et al. [22]. Pelleted chloroplasts were resuspended in the corresponding extraction buffer (see below) and used directly for the enzymatic measurements. *Glycolate oxidase:* Leaf material was homogenized in the presence of N2 and resuspended in extraction buffer consisting in 100 mM Hepes, pH 7.2, 1 mM EDTA and 10 mM 2-mercaptoethanol. The homogenate was clarified by centrifugation and aliquots of 10 µl were used for the enzymatic assays following the method of Yamaguchi and Nishimura [36] with the following modifications: the reaction media contained 100 mM triethanolamine, pH 7.8, 3 mM EDTA, 0.75 mM oxidized glutathione and 4 mM phenylhydrazine. The reaction was started by the addition of 2.3 mM sodium glycolate and followed spectrophotometrically at 320 mm. One unit is defined as the amount of enzyme catalyzing the production of 1 µmol glyoxylate-phenylhydrazone per minute calculated from the extinction coefficient for phenylhydrazon at 324 nm of 16.8 mM-1 cm-1. *Malate synthase:* Leaf material was homogenized in the presence of N2 and resuspended in extraction buffer consisting in 50 mM Tris-HCl, pH 8.0 and 1 mM MgCl₂. The 20 homogenate was clarified by centrifugation and aliquots of 10 µl were used for the enzymatic assays. MS activity was determined following the procedure described by Smith et al. [37]. The reaction media contained 50 mM Tris-HCl, pH 8.0, 5 mM MgCl₂, 3 mM acetyl-CoA, 1,6 mM 5,5'-dithiobis 2-nitrobenzoic acid (DTNB). The reaction was started by the addition of 4 mM glyoxylate and followed spectrophotometrically at 410 mm at 25°C. One unit is defined as the amount of enzyme catalyzing the production of 1 µmol 4-nitrothiolate per minute calculated from the extinction coefficient for 4- nitrothiolate at 410 nm of 13.7 M-1 cm-1. *Catalase*: Leaf material was homogenized in the presence of N2 and resuspended in extraction buffer consisting in 50 mM KH₂PO₄, pH 7.0, 1 % (v/v) polyvinylpyrrolidone (PVP)-40 and 0.1% (v/v) Triton X-100. Catalase activity was determined in 50 mM KH₂PO₄, pH 7.0 as described by Havir and McHale [38]. The reaction was started by the addition of 10 mM H₂O₂ and followed at 240 nm at 25°C. One unit is defined as the amount of enzyme catalyzing the decomposition of 1 µmol H₂O₂ per minute calculated from the extinction coefficient for H₂O₂ at 240 nm of 43.6 M-1 cm-1.

### Example 6: Measurements of H₂O₂ and glyoxylate content

*Hydrogen Peroxide:* H₂O₂ content was determined following a modified version of the protocol described by Okuda et al. [39]. Leaves (50-100 mg) were homogenized in the presence of N₂ and 500 µl of 0.2 M HClO₄ and centrifuged at 20,000g for 5 min at 4°C. To remove HClO₄, 300 µl of the supernatant was neutralized to pH 7.5 with 4 N KOH and the solution was centrifuged at 1,000g for 1 min at 4°C. 200 µl of supernatant was applied to a 1.2 ml column of AG-1 (0.8 cm x 4 cm; BioRad, Hercules, USA), and the column was washed two times with 800 µl of distilled water. The second eluate containing the H₂O₂ was used for the assay. The reaction mixture contained 500 µl of the eluate, 200 µl p-dimethylaminobenzalsehyde (DMAB, 12.5 mM in 0.375 M phosphate buffer, pH 6.5), 40 µl 3-methyl-2-benzothiazolinone hydrazone (MBTH, 0.05% w/v) and 10 µl peroxidase (12.5 U/ml) in a total volume of 750 µl. The reaction was started by the addition of peroxidase and after 8 min incubation at 25°C the increase of absorbance at 590 nm was monitored. *Glyoxylate:* The determination of glyoxylate was conducted using a modification of the protocol described by Häusler et al. [40]. Leaf material (30-100 mg) was homogenized in the presence of N₂ and 500 µl 100 mM HCl and 0.1% phenylhydrazine and incubated at 80°C for 5 min. After chilling on ice, the samples were centrifuged at 10,000g for 2 min and 200 µl of the supernatant was mixed with 750 µl of 18.5% HCl and 50 µl of 4% (w/v) K₃Fe(CN)₆. The mixture was centrifuged at 10,000g for 2 min and the absorbance of the supernatant was measured at 520 nm exactly 8 min after the addition of K₃Fe(CN)₆. As a control, K₃Fe(CN)₆ was omitted.

### Example 7: Metabolite contents

GC-MS analysis, carbohydrate and chlorophyll contents were conducted as described by Fahnenstich et al. [33].

### Example 8: Chlorophyll fluorescence parameters

Chlorophyll fluorescence measurements were performed with a PAM-2000 pulse amplitude modulated chlorophyll fluorometer (Walz GmbH, Effeltrich, Germany). At the start of each measurement, a plant was dark-adapted for 10 min. Basal fluorescence (F0) was measured with modulated weak red light and maximal fluorescence (Fm) was induced with a saturating white light pulse (5000 µmol m-2 sec-1; duration 0.8 s).

### Example 9: CO₂ assimilation rate and carbon isotope analysis

Photosynthetic rate measurements were made on plants that were grown under a 8hlight/ 16h-dark regime at photosynthetically active photon flux densities (PPFD) of 100 and 300 µmol quanta m-2 s-1 (normal and elevated light intensities, respectively) at ambient relative humidity levels and at 24°C, following the procedures of Awada et al., (41). Briefly, the maximum net photosynthesis (A, µmol m-2 s-1) at light saturation were made using a portable photosynthetic system mounted with a LED light source and a leaf chamber specifically constructed for Arabidopsis leaves (LI 6400-2B, LICOR Inc., Lincoln, NE, USA) Carbon isotope ratio measurements were performed according to the procedures of Madhavan et al. [42]. For the determination of carbon isotope ratios, whole rosettes were dried at 60°C for 48 h and ground to a fine powder. A sub-sample of this leaf powder (2-3 mg) was analyzed for carbon isotope ratio using an elemental analyzer (Heraeus, CHN-O Rapid) interfaced with an automated trapping box system and a Finnigan Delta-S isotope ratio mass spectrometer. The isotope ratio of each sample was determined by comparison with a previously calibrated working standard with a known δ13C value relative to PDB (Pee Dee Belemnite international standard).

### Example 10: Co-expression of glycolate oxidase, malate synthase and catalase in A. thaliana chloroplasts

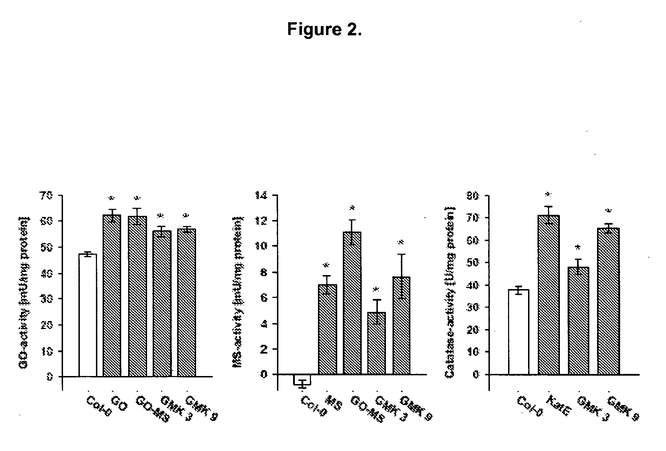

*A. thaliana* was transformed with a plasmid encoding glycolate oxidase (GO). Kanamycin-resistant transgenic lines with GO activities higher than the wild-type (19 to 53%) and containing only one GO transgene insertion were subjected to two more rounds of screening to obtain nonsegregating T3 transgenic lines. A GO representative line with a 30% higher GO activity compared to the wild-type (Figure 2) was transformed with a plasmid encoding malate synthase (MS). Hygromycin-resistant GO-MS transformants were selected and analysed for MS activity. As all transgenic lines obtained showed similar MS activities (9.8 ± 2.2 to 17.9 ± 0.5 mU/mg protein), a representative line (Figure 2) bearing only one MS transgene insertion as revealed by Southern blot, was subjected to two more rounds of screening to obtain a nonsegregating T3 population. This double homozygous overexpressing line was transformed with a plasmid encoding *E. coli* catalase (KatE) and eleven independent lines (GMK lines) were selected by BASTAresistance. GMK lines containing catalase activities higher than the wild-type were subjected to two more rounds of screening to obtain nonsegregating T3 lines. The lines GMK3 and GMK9 were selected for further analysis as representative of the two different populations obtained (see below; Figure 2). Southern blot analysis indicated that the line GMK3 contained only one KatE transgene insertion while GMK9 contained three insertions (not shown). The GMK plants presented GO and MS activities that resembled the parental lines GO and GO-MS (Figure 2), indicating no loss of transgene expression after nine generations. To exclude any phenotypic alteration caused by the single transgene expression, MS and KatE were also expressed in the wild-type background. In both cases, lines with MS or catalase activities similar to those found in the GMK lines were obtained (representative lines are included in Figure 2). Enzymatic measurements performed with isolated chloroplasts from leaves of 5-week-old GMK3 plants and wildtype plants demonstrated that the transgene enzymes were correctly localized to plastids (not shown).

### Example 11: Transgenic GMK plants accumulate more biomass

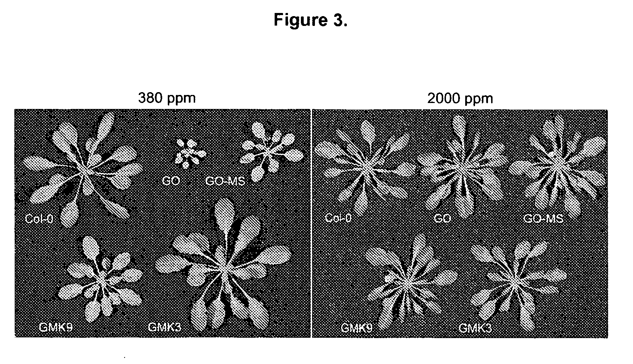

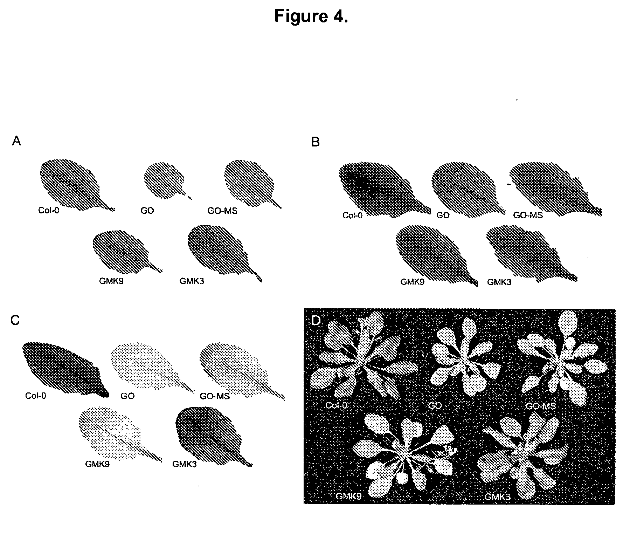

Homozygous GO plants were characterized by a reduced rosette diameter and fresh weight and yellowish leaves during the first seven weeks of growth (Figure 3). Interestingly from the seventh week on, the differences in growth with respect to the wild-type became less pronounced (not shown). Measurements of glyoxylate and water peroxide contents indicated that both were increased in the GO plants (Figure S1). The GO-MS lines resembled the GO ones but with an intermediate size between the GO and the wild-type plants (Figure 3). As the GO-MS plants presented the GO phenotype when exposed to stress conditions (see below), the KatE gene was expressed in the plastids of the GO-MS plants to promote the detoxification of excess H₂O₂ produced by the GO activity. Independent lines presenting all three transgenic activities showed a statistically significant increase in biomass (e. g. GMK3) while others were bigger than the parental GO-MS plants but still smaller than the wild-type (e. g. GMK9). As shown in table 1, GMK3 plants showed an increased rosette diameter accompanied by a higher number of leaves and an increase in the dry weight by 35 to 40% compared to the wild-type. Negligible phenotypical differences were observed when all these lines were grown under non-photorespiratory conditions (CO2 concentration of 2000 ppm (Figure 3 and Table 1). Obviously, the inhibition of the oxygenase reaction of RubisCO led to a reduced production of glycolate inside the chloroplast and, consequently, also to reduced glyoxylate and H₂O₂ levels. It is worth mentioning that the single MS and KatE overexpressing lines behaved like the wild-type under all conditions tested (not shown). We then evaluated the capacity of the transformants to accumulate the end products of photosynthesis at the end of the light period. Sucrose and the monosaccharides glucose and fructose were determined by GC-MS. At ambient CO₂ and in the absence of stress, the GO-MS plants accumulated less sucrose, while the GMK lines showed levels comparable to the wild-type. While no differences in the accumulation of glucose and fructose were observed in the GO-MS plants compared to the wild-type, the levels in GMK lines were higher than those of the wild-type. After exposure to six hours of high light, all these metabolites were decreased in the GO-MS plants, whilst GMK lines accumulated these photoassimilates (Table 2). Likewise, under normal light conditions the GO-MS transformants accumulated less transitory starch (also observed in the parental GO line), while the GMK plants recovered the capacity to accumulate starch as it is the case for the wild-type (Figure 4A). Similar high levels of starch accumulation were observed in all lines when they were grown at high CO₂ concentration (Figure 4B). Interestingly, when the lines were grown under normal conditions and then transferred for seven days to high light conditions, the GMK3 plants accumulated starch like the wild-type, while the GO, GO-MS and GMK9 transgenic lines have lost this capacity (Figure 4D). In line with these results, the GO, GO-MS and GMK9 lines showed intense yellowing and oxidative lesions in response to high light treatment (Figure 4D).

### Example 12: Enhanced CO₂ assimilation and reduced photorespiration in GMK plants

To evaluate the photosynthetic performance of the transgenic plants, chlorophyll fluorescence, gas exchange and discrimination against ¹³C (carbon isotope ratio, δ¹³C) were determined in the different genotypes. The electron transport rate (ETR) of the GO plants was diminished by approximately 25% in comparison to the wild-type, while those of the GO-MS and GMK9 plants was similar to that of the wild-type (Table 3). In the case of the GMK3 plants, the ETR was enhanced by 26% compared to the wild-type (Table 3). The F_{V}/F_{M} ratio indicated that the maximum quantum efficiency of photosystem II was not affected in 7-week-old GO-MS and GMK plants grown under normal conditions, while the GO plants showed a slightly negative effect (Table 3). It is worth mentioning that younger GO plants always presented much lower F_{V}/F_{M} ratios than the other lines, a fact that correlated well with the more pronounced visual phenotype of these plants during the first weeks of growth (not shown). On the other hand, when the plants were exposed to high light for six hours, no differences in the F_{V}/F_{M} ratio was observed in the GMK lines compared to the wild-type, while a strong photoinhibition was observed in GO and GO-MS plants (not shown). Instantaneous photosynthetic rate measurements (A, µmol m⁻² s⁻¹) were made on both control and transformants expressing the whole pathways using a LICOR portable photosynthesis meter. Table 3 shows that the photosynthetic rate of the GMK3 line was relatively higher than the wild-type, while the one of line GMK9 was comparable to the wild-type. To determine if GMK3 plants are photosynthetically more efficient than their parental lines, carbon isotope fractionation studies were performed. Carbon isotope ratio measurements provide an integrated view of photosynthetic function performed of plants throughout their life span. As shown in Table 3, when plants were grown under normal conditions, the δ¹³C were less negative for the GMK3 line compared to the wild-type (a similar result was obtained for the GMK1 line), indicating increased carboxylation efficiency and water use efficiency in these plants. As anticipated, the δ¹³C of all lines showed no differential trend when the plants were grown under non-photorespiratory conditions (2,000 ppm CO₂, Table 3). Glycine and serine levels were determined by GC-MS and the Gly/Ser ratio was calculated as an indicator of flux through the photorespiratory pathway. The GMK lines presented lower glycine contents than the wild-type at ambient CO2 concentrations and under normal or high light conditions (Table 3). The GO-MS plants showed invariable glycine contents under normal light conditions but showed decreased levels under high light (Table 3). In the case of serine, the GMK plants showed lower levels than the wildtype under both light intensities tested, while the GO-MS plants showed wild-type levels under normal light and increased serine levels under high light (Table 3). The Gly/Ser ratio of all lines was comparable under normal light conditions but showed significantly lower ratios determined for the transgenic lines after six hours at high light intensities compared to the wild-type (Table 3). After incubating the plants under this photorespiratory condition, the Gly/Ser ratio of the wild-type increased about 150-fold compared to normal light conditions due to a high accumulation of glycine (Table 3). This increase was only 33, 13- and 63-fold in the case of GO-MS, GMK3 and GMK9, respectively (Table 3). The GMK3 plants presented the lowest Gly/Ser ratio due to a very low glycine accumulation in these plants. No differences in the Gly/Ser ratio were observed between the lines when grown at high CO₂ concentrations (Table 3).

**Table 1. Growth parameters of 8-week-old transgenic and wild-type lines. Rosette diameter (RD), number of leaves (NL) and dry weight (DW) of plants grown in short days at ambient (A, 380 ppm) or at elevated CO₂ (E, 2,000 ppm) concentrations. The values presented are the means ± SE of two replicates of pools of at least eight plants each. The values set in bold case indicate significant differences to the wild-type values calculated by the Student's test (P<0.05).**

| | | wild-type | GO-MS | GMK3 | GMK9 |
|---|---|---|---|---|---|
| RD (cm) | A | 10.5 ± 0.2 | 9.4 ± 0.3 | **12.1 ± 0.2** | 9.4 ± 0.3 |
| NL | A | 33.3 ± 0.8 | 33.4 ± 0.5 | **39.5 ± 0.7** | 32.2 ± 0.9 |
| | E | 31.0 ± 0.4 | 32.0 ± 0.6 | 31.1 ± 0.6 | 32.1 ± 0.5 |
| DW (mg) | A | 106.9 ± 5.6 | 106.7 ± 6.0 | **146.6 ± 3.9** | 104.3 ± 8.3 |
| | E | 184.7 ± 16.1 | 185.4 ± 7.9 | 199.7 ± 8.4 | 199.6 ± 5.9 |

**Table 2. Photosynthate levels measured by GC-MS of whole rosette leaves harvested after 6 hours in the light at different light intensities (L: low light (30 µmol m⁻² s⁻¹); H: high light (600 µmol m⁻² s⁻¹)). The values presented are the means ± SE of two replicates of pools of at least eight plants each. The values set in bold case indicate significant differences to the wild-type values calculated by the Student's test (P<0.05).**

| | wild-type | | GO-MS | | GMK3 | | GMK9 | |
|---|---|---|---|---|---|---|---|---|
| | L | H | L | H | L | H | L | H |
| Glucose | 8.43 ± 1.48 | 122.5 ± 4.6 | 11.49 ± 2.36 | **35.80 ± 0.50** | **12.13 ± 0.65** | **86.8 ± 8,6** | 10.87 ± 0.64 | 103.5 ± 2.5 |
| Fructose | 1.65 ± 0.25 | 10.30 ± 0.51 | 1.63 ± 0.18 | **3.27 ± 0.05** | **3.11 ± 0.03** | 8.83 ± 0.64 | 2.28 ± 0.18 | 16.27 ± 0.90 |
| Sucrose | 34.90 ± 1.90 | 338.7 ± 16.7 | **24.50 ± 2.30** | **160.9 ± 6.1** | 36.30 ± 2.70 | 173.5 ± 3.10 | 33.80 ± 0.70 | 236.2 ± 8.2 |
| Glycine | 0.67 ± 0.03 | 59.62 ± 7.20 | 0.68 ± 0.04 | **18.50 ± 0.90** | **0.51 ± 0.08** | **9.50 ± 1.90** | **0.35 ± 0.14** | **28.10 ± 5.10** |
| Serine | 16.80 ± 1.20 | 9.70 ± 1.40 | 18.80 ± 1.20 | **15.50 ± 0.40** | **6.70 ± 1.80** | **5.90 ± 0.50** | **9.00 ± 2.80** | **7.6 ± 1.50** |

**Table 3. Photosynthetic performance of wild-type and the transgenic lines. A, CO₂ assimilation rate at 380 ppm CO₂ ETR, Electron Transport Rate determined at 600 µmol m⁻² s⁻¹; F_{V}/F_{M}, maximum quantum efficiency of photosystem II determined after six hours at 600 µmol m⁻² s⁻¹; Δδ ¹³C, Differences in whole leaf δ¹³C values of the transformants when compared to the wild-type; Gly/Ser, Glycine to serine ratio. The values presented are the means ± SE of two replicates of pools of at least eight plants each and those set in bold case indicate significant differences to the wild-type values calculated by the Student's test (P<0.05). If not stated otherwise, the measurements were conducted with plants grown under normal light intensities. n.d.: not determined.**

| | Wild-type | GO | GO-MS | GMK3 | GMK9 |
|---|---|---|---|---|---|
| **A** (µmol m⁻² s⁻¹) | 8.23 ± 0.21 | n.d. | n.d. | **9.16 ± 0.17** | 8.72 ± 0.18 |
| **ETR** (µmol m⁻² s⁻¹) | 70.5 ± 2,7 | **52.9 ± 9.8** | 74.6 ± 4.1 | **89.1 ± 3.4** | 75.8 ± 2.6 |
| F_{V}/F_{M} | 0.68 ± 0.02 | **0.54 ± 0.02** | **0.55 ± 0.01** | 0.69 ± 0.04 | 0.65 ± 0.05 |
| Δδ¹³C | | | | | |
| at 380 ppm | | n.d. | n.d. | 0.87 ± 0.01 | - 0.11 ± 0.07 |
| at 2,000 ppm | | n.d. | n.d. | - 0.04 ± 0.02 | - 0.19 ± 0.08 |
| **Gly/Ser** | | | | | |
| at 380 ppm (30 µmol m⁻² s⁻) | 0.040 ± 0.001 | 0.05 ± 0.015 | 0.037 ± 0.001 | 0.050 ± 0.011 | 0.032 ± 0.009 |
| at 380 ppm (600 µmol m⁻² s⁻) | 6.238 ± 0.206 | **1.235 ± 0.049** | **1.189** ± **0.039** | **1.574 ± 0.235** | **3.807 ± 0.382** |
| at 2,000 ppm | 0.035 ± 0.001 | 0.032 ± 0.001 | 0.039 ± 0.003 | 0.043 ± 0.004 | 0.040 ± 0.004 |

References as far as not mentioned explicitly in the description are found in the following list of references:

### REFERENCES

1. Tolbert NE (1997) The C2 oxidative photosynthetic carbon cycle. Annu. Rev. Plant Physiol. Plant Mol. Biol. 48: 1-25.
2. Douce R and Neuburger M (1999) Biochemical dissection of photorespiration. Curr. Opin. Plant Biol. 2: 214-222.
3. Wingler A, Lea PJ, Quick WP, Leegood RC (2000) Photorespiration: metabolic pathways and their role in stress protection. Phil. Trans. R. Soc. Lond B 255: 1517- 1529.
4. Keys AJ, Bird IF, Cornelius MJ, Lea PJ, Wallsgrove RM, Miflin BJ (1978) Photorespiratory nitrogen cycle. Nature 275: 741-743.
5. Leegood RC, Lea PJ, Adcok MD, Häusler RE (1995) The regulation and control of photorespiration. J. Exp. Bot. 46: 1397-1414.
6. Parry M, Andralojc PC, Mitchell RAC, Madgwick P, Keys A (2003) Manipulation of RubisCO: the amount, activity, function and regulation. J. Exp. Bot. 54: 132-1333.
7. Somerville CR, Ogren WL (1980) Photorespiration mutants of Arabidopsis thaliana deficient in serine-glycolate aminotransferase activity. Proc. Natl. Acad. Sci. USA 77: 2684-2687.
8. Somerville CR, Ogren WL (1980) Inhibition of photosynthesis in Arabidopsis mutants lacking leaf glutamate synthase activity. Nature 286: 257-259.
9. Somerville CR, Ogren WL (1981) Photorespiration-deficient mutants of Arabidopsis thaliana lacking mitochondrial serine transhydroxymethylase activity. Plant Physiol. 67: 666-671.
10. Somerville CR, Ogren WL (1982) Mutants of the cruciferous plant Arabidopsis thaliana lacking glycine decarboxylase activity. Biochem. J. 202: 373-380. 25
11. Sommerville S, Somerville CR (1985) Mutant of Arabidopsis thaliana deficient in chloroplast dicarboxylate transport is missing an envelope protein. Plant Sci. Lett. 37: 217-220.
12. Bauwe H. and Kolukisaoglu U. (2003) Genetic manipulation of glycine decarboxylation. J Exp. Bot. 54: 1523-1535.
13. Bold R, Edner C, Kolukisaoglu U, Hagemann M, Weckwerth W, et al. (2005) DGlycerate 3-kinase the last unknown enzyme in the photorespiratory cycle in Arabidopsis, belongs to a novel kinase family. Plant Cell 17: 2413-2420.
14. Bagder MR, Price GD (2003) CO2 concentrating mechanisms in cyanobacteria: molecular components, their diversity and evolution. J. Exp. Bot. 54: 609-622.
15. Giordano M, Beardall J, Raven JA (2005) CO2 concentrating mechanisms in algae: mechanisms, environmental modulation and evolution. Ann. Rev. Plant Biol. 56, 99- 131.
16. Hatch MD (1987) C4 photosynthesis: A unique bend of modified biochemistry, anatomy and ultrastructure. Biochim. Biophys. Acta 895: 8-16.
17. Sage RF, Pearcy RW, Seemann JR (1987) The nitrogen use efficiency of C3 and C4plants. Plant Physiol. 85: 355-359.
18. Ishimaru K, Ichikawa H, Matsuoka M, Oshugi R (1997) Analysis of a C4 pyruvate, orthophosphate dikinase expressed in C3 transgenic Arabidopsis plants. Plant Sci. 129: 57-64.
19. Ishimaru K, Ohkawa Y, Ishige T, Tobias DJ, Oshugi R (1998) Elevated pyruvate orthophosphate dikinase (PPDK) activity alters carbon metabolism in C3-transgenic potatoes with a C4 maize PPDK gene. Physiol. Plant. 103: 340-346.
20. Matsuoka M, Furbank RT, Fukayama H, Miyao M (2001) Molecular engineering of C4 photosynthesis. Ann. Rev. Plant Physiol. Plant Mol. Biol. 52: 297-314.
21. Häusler RE, Rademacher T, Li J, Lipka V, Fischer KL, et al. (2001) Single and double overexpression of C4-cycle genes had differential effects on the pattern of endogenous enzymes, attenuation of photorespiration and on contents of UV protectants in transgenic potato and tobacco plants. J. Exp. Bot. 52: 1785-1803.
22. Kebeish R, Niessen N, Thiruveedhi K, Bari R, Hirsch J-H, et al. (2007) Chloroplastic photorespiratory bypass increases photosynthesis and biomass production in Arabidopsis thaliana. Nature Biotech. 25: 593-599.
23. Keys AJ (1999) Biochemistry of photorespiration and the consequences for plant performance. In Plant Carbohydrate Biochemistry (eds. JA Bryant, MM Burell, NJ Kruger), pp. 147-162. Oxford: BIOS Scientific Publishers.
24. Noctor G, Arisi A-CM, Jouanin L, Foyer CH (1999) Photorespiratory glycine enhances glutathione accumulation in both the chloroplastic and cytosolic compartments. J. Exp. Bot. 50: 1157-1167.
25. Heber U, Bligny R, Streb P, Douce R (1996) Photorespiration is essential for protection of the photosynthetic apparatus of C3-plants against photoinactivation under sunlight. Bot. Acta. 109: 307-315.
26. Kozaki A, Takeba G (1996) Photorespiration protects C3-plants from photooxidation. Nature 384, 557-560.
27. Farquhar GD, Ehleringer JR, Hubick KT (1989) Carbon isotope discrimination and photosynthesis. Ann. Rev. Plant Physiol. 40: 503-537.
28. Gillon JS, Griffiths H (1997) The influence of (photo)respiration on carbon isotope discrimination in plants. Plant Cell Environ. 20:1217-1230.
29. Igamberdiev AU, Mikkelsen T, Ambus P, Bauwe H, Lea P, et al. (2004) Photorespiration contributes to stomatal regulation and carbon isotope fractionation: a study with barley, potato and Arabidopsis plant deficient in glycine decarboxylase. Photos Res. 81: 139-152.
30. von Cammerer S (2003) C4 photosynthesis in a single cell is theoretically inefficient but may ameliorate internal CO2 diffusion limitations of C3 leaves. Plant Cell Environm. 26: 1191-1197.
31. Horng J-T, Behari R, Burke C-A, Baker A (1995) Investigation of the Energy Requirement and Targeting Signal for the Import of Glycolate Oxidase into Glyoxysomes. Eur. J. Biochem. 230: 157-163.
32. Mori H, Takeda-Yoshikawa Y, Hara-Nishimura I, Nishimura M (1991) Pumpkin malate synthase Cloning and sequencing of the cDNA and Northern blot analysis. Eur. J. Biochem. 197: 331-336.
33. Fahnenstich H, Saigo M, Niessen M, Zanor MI, et al. (2007) Alteration of organic acid metabolism in Arabidopsis thaliana overexpressing the maize C4-NADP-malic enzyme causes accelerated senescence during extended darkness. Plant Physiol. 145: 640-652.
34. Hellens RP, Edwards EA, Leyland NR, Bean S, Mullineaux PM (2000) pGreen: a versatile and flexible binary Ti vector for Agrobacterium-mediated plant transformation. Plant Mol. Biol. 42: 819-832.
35. Bechtold, N, Ellis J and Pelletier, G (1993) In planta Agrobacterium-mediated gene transfer by infiltration of adult Arabidopsis thaliana plants. C R Acad Sci Paris, Life Sci. 316: 1194-1199.
36. Yamaguchi K and Nishimura M (2000) reduction to below threshold levels of glycolate oxidase activities in transgenic tobacco enhances photoinhibition during irradiation. Plant Cell Physiol. 41: 1397-1406.
37. Smith CV, Huang C-C, Miczak A, Russell DG, Sacchettini JC, et al. (2003) Biochemical and Structural Studies of Malate Synthase from Mycobacterium tuberculosis. J. Biol. Chem. 278: 1735-1743.
38. Havir E, McHale N (1987) Biochemical and developmental characterization of multiple forms of catalase in tobacco leaves. Plant Physiol. 84: 450-455.
39. Okuda T, Matsuda Y, Yamanaska A, Sagisaka S (1991) Abrupt increase in the level of hydrogen peroxide in leaves of winter wheat is caused by cold treatment. Plant Physiol. 97: 1265-1267.
40. Häusler RE, Bailey KJ, Lea PJ, Leegood RC (1996) Control of photosynthesis in barley mutants with reduced activities of glutamine synthetase and glutamate synthase. Planta 200: 388-396.
41. Awada T, Dunigan DD, Dickman MB (2004) Animal anti-apoptotic genes enhance recovery from drought stress in tobacco. Int. J. Agri & Biol. 6: 943-949.
42. Madhavan S, Treichel I, O'Leary MH (1991) Effects of relative humidity on carbon isotope fractionation in plants. Bot. Acta 104: 292-294.

## Claims

1. A plant cell comprising in its chloroplasts enzymatic activities for converting glycolate into malate, wherein said plant cell comprises in its chloroplasts a first polypeptide having glycolat oxidase activity, a second polypeptide having malate synthase activity and a third polypeptide having catalase activity.

2. The plant cell of claim 1, wherein said first polypeptide is encoded by a first polynucleotide comprising a nucleic acid selected from the group consisting of:
a. a nucleic acid having a nucleotide sequence as shown in SEQ ID No: 1;
b. a nucleic acid encoding a polypeptide having an amino acid sequence as shown in SEQ ID No: 2;
c. a nucleic acid having a nucleotide sequence being at least 50% identical to the nucleotide sequence shown in SEQ ID No: 1 wherein said nucleic acid encodes a polypeptide having glycolate oxidase activity; and
d. a nucleic acid encoding a polypeptide having an amino acid sequence being at least 50% identical to the amino acid sequence shown in SEQ ID No:2, wherein said polypeptide has glycolate oxidase activity.

3. The plant cell of claim 1 or 2, wherein said second polypeptide is encoded by a second polynucleotide comprising a nucleic acid selected from the group consisting of:
a. a nucleic acid having a nucleotide sequence as shown in SEQ ID No: 3;
b. a nucleic acid encoding a polypeptide having an amino acid sequence as shown in SEQ ID No: 4;
c. a nucleic acid having a nucleotide sequence being at least 40% identical to the nucleotide sequence shown in SEQ ID No: 3 wherein said nucleic acid encodes a polypeptide having malate synthase activity; and
d. a nucleic acid encoding a polypeptide having an amino acid sequence being at least 40% identical to the amino acid sequence shown in SEQ ID No: 4, wherein said polypeptide has malate synthase activity.

4. The plant cell of any one of claims 1 to 3, wherein third polypeptide is encoded by a third polynucleotide comprising a nucleic acid selected from the group consisting of:
a. a nucleic acid having a nucleotide sequence as shown in SEQ ID No: 5;
b. a nucleic acid encoding a polypeptide having an amino acid sequence as shown in SEQ ID No: 6;
c. a nucleic acid having a nucleotide sequence being at least 70% identical to the nucleotide sequence shown in SEQ ID No: 5 wherein said nucleic acid encodes a polypeptide having catalase activity; and
d. a nucleic acid encoding a polypeptide having an amino acid sequence being at least 50% identical to the amino acid sequence shown in SEQ ID No: 6, wherein said polypeptide has catalase activity.

5. The plant cell of any one of claims 1 to 4 wherein said first polypeptide is expressed from a heterologous polynucleotide.

6. The plant cell of claim 5, wherein said heterologous polynucleotide comprises a nucleic acid as defined in claim 2.

7. The plant cell of any one of claims 1 to 6, wherein said second polypeptide is expressed from a heterologous polynucleotide.

8. The plant cell of claim 7, wherein said heterologous polynucleotide comprises a nucleic acid as defined in claim 3.

9. The plant cell of any one of claims 1 to 8, wherein said third polypeptide is expressed from a heterologous polynucleotide.

10. The plant cell of any one of claim 9, wherein said heterologous polynucleotide comprises a nucleic acid as defined in claim 4.

11. The plant cell of any one of claims 1 to 10, wherein said plant cell further comprises an NADP-malic enzyme and a pyruvate dehydrogenase.

12. The plant cell of any one of claims 1 to 11, wherein said plant cell has an increased efficiency of CO₂ assimilation compared to a plant cell lacking enzymatic activities for converting glycolate into malate.

13. The plant cell of any one of claim 1 to 12, wherein said plant cell is a cell of a C₃ plant.

14. A plant comprising the plant cell of any one of claims 1 to 13.

15. The plant of claim 14, wherein said plant has increased CO₂ assimilation compared to a plant lacking a plant cell of any one of claims 1 to 13.

16. The plant of claim 14 or 15, wherein said plant has an attenuated photorespiration compared to a plant lacking a plant cell of any one of claims 1 to 13

17. The plant of any one of claims 14 to 16, wherein said plant has increased water use efficiency compared to a plant lacking a plant cell of any one of claims 1 to 13

18. The plant of any one of claims 14 to 17, wherein said plant produces an increased number of leaves compared to a plant lacking a plant cell of any one of claims 1 to 13.

19. The plant of any one of claims 14 to 18, wherein said plant has an increased yield compared to a plant lacking a plant cell of any one of claims 1 to 13.

20. A seed obtainable from a plant of any one of claims 14 to 19 wherein said seed is capable of generating a plant of any one of claims 14 to 19.

21. A method for producing a transgenic plant or a plant cell having an increased water use efficiency compared to a corresponding non-transgenic plant or plant cell, said method comprises introducing in the chloroplasts of the transgenic plant or plant cell a first polypeptide, a second polypeptide and a third polypeptide as defined in any one of claims 1 to 10, wherein said transgenic plant or plant cell is produced by transformation.

22. A method for producing a transgenic plant or plant cell having increased yield compared to a corresponding non-transgenic plant or plant cell, said method comprises introducing into the chloroplasts of the transgenic plant or plant cell a first polypeptide, a second polypeptide and a third polypeptide as defined in any one of claims 1 to 10, wherein said transgenic plant or plant cell is produced by transformation.

23. The method of claims 21 or 22, wherein said method comprises the steps of:
a. introducing at least one heterologous polynucleotide encoding the said polypeptides into the plant of plant cell; and
b. expressing said polypeptides from the said at laest one polynucleotide.

24. A polynucleotide comprising in combination a nucleic acid as defined in claim 2, a nucleic acid as defined in claim 3 and a nucleic acid as defined in claim 4.

25. A vector comprising the polynucleotide of claim 24.

26. The vector of claim 25, wherein said vector is an expression vector.

27. A composition comprising a first polynucleotide as defined in claim 2, a second polynucleotide as defined in claim 3 and a third polynucleotide as defined in claim 4.

28. Use of the polynucleotide of claim 24, the vector of claim 25 or the composition of claim 27 for conferring increased water use efficiency to a plant or plant cell.

29. Use of the polynucleotide of claim 24, the vector of claim 25 or the composition of claim 27 for conferring increased yield to a plant or plant cell.

## Patentansprüche

1. Pflanzenzelle, die in ihren Chloroplasten enzymatische Aktivitäten zum Umwandeln von Glycolat in Malat umfasst, wobei die Pflanzenzelle in ihren Chloroplasten ein erstes Polypeptid mit einer Glycolatoxidaseaktivität, ein zweites Polypeptid mit einer Malatsynthaseaktivität und ein drittes Polypeptid mit einer Katalaseaktivität umfasst.

2. Pflanzenzelle nach Anspruch 1, wobei das erste Polypeptid durch ein erstes Polynukleotid codiert wird, das eine Nukleinsäure umfasst, ausgewählt aus der Gruppe, bestehend aus:
a. einer Nukleinsäure mit einer Nukleotidsequenz nach SEQ ID NO: 1;
b. einer Nukleinsäure, die ein Polypeptid mit einer Aminosäuresequenz nach SEQ ID NO: 2 codiert;
c. einer Nukleinsäure mit einer Nukleotidsequenz, die zu der Nukleotidsequenz nach SEQ ID NO: 1 mindestens 50% identisch ist, wobei die Nukleinsäure ein Polypeptid mit einer Glycolatoxidaseaktivität codiert; und
d. einer Nukleinsäure, die ein Polypeptid mit einer Aminosäuresequenz codiert, die zu der Aminosäuresequenz nach SEQ ID NO: 2 mindestens 50% identisch ist, wobei das Polypeptid eine Glycolatoxidaseaktivität aufweist.

3. Pflanzenzelle nach Anspruch 1 oder 2, wobei das zweite Polypeptid durch ein zweites Polynukleotid codiert wird, das eine Nukleinsäure umfasst, ausgewählt aus der Gruppe, bestehend aus:
a. einer Nukleinsäure mit einer Nukleotidsequenz nach SEQ ID NO: 3;
b. einer Nukleinsäure, die ein Polypeptid mit einer Aminosäuresequenz nach SEQ ID NO: 4 codiert;
c. einer Nukleinsäure mit einer Nukleotidsequenz, die zu der Nukleotidsequenz nach SEQ ID NO: 3 mindestens 40% identisch ist, wobei die Nukleinsäure ein Polypeptid mit einer Malatsynthaseaktivität codiert; und
d. einer Nukleinsäure, die ein Polypeptid mit einer Aminosäuresequenz codiert, die zu der Aminosäuresequenz nach SEQ ID NO: 4 mindestens 40% identisch ist, wobei das Polypeptid eine Malatsynthaseaktivität aufweist.

4. Pflanzenzelle nach einem der Ansprüche 1 bis 3, wobei das dritte Polypeptid durch ein drittes Polynukleotid codiert wird, das eine Nukleinsäure umfasst, ausgewählt aus der Gruppe, bestehend aus:
a. einer Nukleinsäure mit einer Nukleotidsequenz nach SEQ ID NO: 5;
b. einer Nukleinsäure, die ein Polypeptid mit einer Aminosäuresequenz nach SEQ ID NO: 6 codiert;
c. einer Nukleinsäure mit einer Nukleotidsequenz, die zu der Nukleotidsequenz nach SEQ ID NO: 5 mindestens 70% identisch ist, wobei die Nukleinsäure ein Polypeptid mit einer Katalaseaktivität codiert; und
d. einer Nukleinsäure, die ein Polypeptid mit einer Aminosäuresequenz codiert, die zu der Aminosäuresequenz nach SEQ ID NO: 6 mindestens 50% identisch ist, wobei das Polypeptid eine Katalaseaktivität aufweist.

5. Pflanzenzelle nach einem der Ansprüche 1 bis 4, wobei das erste Polypeptid von einem heterologen Polynukleotid exprimiert wird.

6. Pflanzenzelle nach Anspruch 5, wobei das heterologe Polynukleotid eine Nukleinsäure, wie in Anspruch 2 definiert, umfasst.

7. Pflanzenzelle nach einem der Ansprüche 1 bis 6, wobei das zweite Polypeptid von einem heterologen Polynukleotid exprimiert wird.

8. Pflanzenzelle nach Anspruch 7, wobei das heterologe Polynukleotid eine Nukleinsäure, wie in Anspruch 3 definiert, umfasst.

9. Pflanzenzelle nach einem der Ansprüche 1 bis 8, wobei das dritte Polypeptid von einem heterologen Polynukleotid exprimiert wird.

10. Pflanzenzelle nach Anspruch 9, wobei das heterologe Polynukleotid eine Nukleinsäure, wie in Anspruch 4 definiert, umfasst.

11. Pflanzenzelle nach einem der Ansprüche 1 bis 10, wobei die Pflanzenzelle zudem ein NADP-Malatenzym und eine Pyruvatdehydrogenase umfasst.

12. Pflanzenzelle nach einem der Ansprüche 1 bis 11, wobei die Pflanzenzelle eine erhöhte Effizienz der CO₂-Assimilation im Vergleich zu einer Pflanzenzelle hat, der die Enyzmaktivitäten zum Umwandeln von Glycolat zu Malat fehlen.

13. Pflanzenzelle nach einem der Ansprüche 1 bis 12, wobei die Pflanzenzelle eine Zelle einer C₃-Pflanze ist.

14. Pflanze, die die Pflanzenzelle nach einem der Ansprüche 1 bis 13 umfasst.

15. Pflanze nach Anspruch 14, wobei die Pflanze eine erhöhte CO₂-Assimilation im Vergleich zu einer Pflanze hat, der eine Pflanzenzelle nach einem der Ansprüche 1 bis 13 fehlt.

16. Pflanze nach Anspruch 14 oder 15, wobei die Pflanze eine abgeschwächte Photorespiration im Vergleich zu einer Pflanze hat, der eine Pflanzenzelle nach einem der Ansprüche 1 bis 13 fehlt.

17. Pflanze nach einem der Ansprüche 14 bis 16, wobei die Pflanze eine erhöhte Wassernutzungseffizienz im Vergleich zu einer Pflanze hat, der eine Pflanzenzelle nach einem der Ansprüche 1 bis 13 fehlt.

18. Pflanze nach einem der Ansprüche 14 bis 17, wobei die Pflanze eine erhöhte Anzahl von Blättern im Vergleich zu einer Pflanze produziert, der eine Pflanzenzelle nach einem der Ansprüche 1 bis 13 fehlt.

19. Pflanze nach einem der Ansprüche 14 bis 18, wobei die Pflanze einen erhöhten Ertrag im Vergleich zu einer Pflanze aufweist, der eine Pflanzenzelle nach einem der Ansprüche 1 bis 13 fehlt.

20. Samen, erhältlich aus einer Pflanze nach einem der Ansprüche 14 bis 19, wobei der Samen eine Pflanze nach einem der Ansprüche 14 bis 19 erzeugen kann.

21. Verfahren zum Herstellen einer transgenen Pflanze oder einer Pflanzenzelle mit einer erhöhten Wassernutzungseffizient im Vergleich zu einer entsprechenden nicht-transgenen Pflanze oder Pflanzenzelle, wobei das Verfahren das Einbringen eines ersten Polypeptids, eines zweiten Polypeptids, und eines dritten Polypeptids, wie definiert in einem der Ansprüche 1 bis 10 in die Chloroplasten der transgenen Pflanze oder Pflanzenzelle umfasst, wobei die transgene Pflanze oder Pflanzenzelle durch Transformation produziert wird.

22. Verfahren zum Herstellen einer transgenen Pflanze oder Pflanzenzelle mit einem erhöhtem Ertrag im Vergleich zu einer entsprechenden nicht-transgenen Pflanze oder Pflanzenzelle, wobei das Verfahren das Einbringen eines ersten Polypeptids, eines zweiten Polypeptids, und eines dritten Polypeptids, wie definiert in einem der Ansprüche 1 bis 10 in die Chloroplasten der transgenen Pflanze oder Pflanzenzelle umfasst, wobei die transgene Pflanze oder Pflanzenzelle durch Transformation produziert wird.

23. Verfahren nach Anspruch 21 oder 22, wobei das Verfahren die folgenden Schritte umfasst:
a. Einbringen von mindestens einem heterologen Polynukleotid, das die Polypeptide codiert, in die Pflanze oder Pflanzenzelle; und
b. Exprimieren der Polynukleotide von dem mindestens einen Polynukleotid.

24. Polynukleotid, umfassend in Kombination eine Nukleinsäure, wie in Anspruch 2 definiert, eine Nukleinsäure, wie in Anspruch 3 definiert, und eine Nukleinsäure, wie in Anspruch 4 definiert.

25. Vektor, umfassend das Polynukleotid nach Anspruch 24.

26. Vektor nach Anspruch 25, wobei der Vektor ein Expressionsvektor ist.

27. Zusammensetzung, umfassend ein erstes Polynukleotid, wie in Anspruch 2 definiert, ein zweites Polynukleotid, wie in Anspruch 3 definiert, und ein drittes Polynukleotid, wie in Anspruch 4 definiert.

28. Verwendung des Polynukleotids nach Anspruch 24, des Vektors nach Anspruch 25 oder der Zusammensetzung nach Anspruch 27 zum Vermitteln einer erhöhten Wassemutzungseffizienz für eine Pflanze oder Pflanzenzelle.

29. Verwendung des Polynukleotids nach Anspruch 24, des Vektors nach Anspruch 25 oder der Zusammensetzung nach Anspruch 27 zum Vermitteln eines erhöhten Ertrags für eine Pflanze oder Pflanzenzelle.

## Revendications

1. Cellule de plante comprenant dans ses chloroplastes des activités enzymatiques permettant de convertir le glycolate en malate, dans laquelle ladite cellule de plante comprend dans ses chloroplastes un premier polypeptide ayant une activité de glycolate oxydase, un deuxième polypeptide ayant une activité de malate synthase, et un troisième polypeptide ayant une activité de catalase.

2. Cellule de plante selon la revendication 1, dans laquelle ledit premier polypeptide est codé par un premier polynucléotide, qui comprend un acide nucléique choisi dans le groupe constitué par :
a. un acide nucléique ayant une séquence nucléotidique telle que présentée dans l'identification de séquence n° 1 ;
b. un acide nucléique codant pour un polypeptide qui a une séquence d'acides aminés telle que présentée dans l'identification de séquence n°2;
c. un acide nucléique ayant une séquence nucléotidique qui est identique, au moins à 50%, à la séquence nucléotidique présentée dans l'identification de séquence n° 1, dans laquelle ledit acide nucléique code pour un polypeptide ayant une activité de glycolate oxydase ; et
d. un acide nucléique codant pour un polypeptide ayant une séquence d'acides aminés qui est identique, au moins à 50%, à la séquence d'acides aminés présentée dans l'identification de séquence n° 2, dans laquelle ledit polypeptide a une activité de glycolate oxydase.

3. Cellule de plante selon la revendication 1 ou la 2, dans laquelle ledit deuxième polypeptide est codé par un deuxième polynucléotide, qui comprend un acide nucléique choisi dans le groupe constitué par :
a. un acide nucléique ayant une séquence nucléotidique telle que présentée dans l'identification de séquence n° 3 ;
b. un acide nucléique codant pour un polypeptide qui a une séquence d'acides aminés telle que présentée dans l'identification de séquence n°4;
c. un acide nucléique ayant une séquence nucléotidique qui est identique, au moins à 40%, à la séquence nucléotidique présentée dans l'identification de séquence n° 3, dans laquelle ledit acide nucléique code pour un polypeptide ayant une activité de malate synthase ;
d. un acide nucléique codant pour un polypeptide ayant une séquence d'acides aminés qui est identique, au moins à 40%, à la séquence d'acides aminés présentée dans l'identification de séquence n° 4, dans laquelle ledit polypeptide a une activité de malate synthase.

4. Cellule de plante selon l'une quelconque des revendications 1 à 3, dans laquelle un troisième polypeptide est codé par un troisième polynucléotide, qui comprend un acide nucléique choisi dans le groupe constitué par :
a. un acide nucléique ayant une séquence nucléotidique telle que présentée dans l'identification de séquence n° 5 ;
b. un acide nucléique codant pour un polypeptide qui a une séquence d'acides aminés telle que présentée dans l'identification de séquence n°6;
c. un acide nucléique ayant une séquence nucléotidique qui est identique, au moins à 70%, à la séquence nucléotidique présentée dans l'identification de séquence n° 5, dans laquelle ledit acide nucléique code pour un polypeptide ayant une activité de catalase ;
d. un acide nucléique codant pour un polypeptide ayant une séquence d'acides aminés qui est identique, au moins à 50%, à la séquence d'acides aminés présentée dans l'identification de séquence n° 6, dans laquelle ledit polypeptide a une activité de catalase.

5. Cellule de plante selon l'une quelconque des revendications 1 à 4, dans laquelle ledit premier polypeptide est exprimé à partir d'un polynucléotide hétérologue.

6. Cellule de plante selon la revendication 5, dans laquelle ledit polynucléotide hétérologue comprend un acide nucléique tel que défini dans la revendication 2.

7. Cellule de plante selon l'une quelconque des revendications 1 à 6, dans laquelle ledit deuxième polypeptide est exprimé à partir d'un polynucléotide hétérologue.

8. Cellule de plante selon la revendication 7, dans laquelle ledit polynucléotide hétérologue comprend un acide nucléique tel que défini dans la revendication 3.

9. Cellule de plante selon l'une quelconque des revendications 1 à 8, dans laquelle ledit troisième polypeptide est exprimé à partir d'un polynucléotide hétérologue.

10. Cellule de plante selon la revendication 9, dans laquelle ledit polynucléotide hétérologue comprend un acide nucléique tel que défini dans la revendication 4.

11. Cellule de plante selon l'une quelconque des revendications 1 à 10, dans laquelle ladite cellule de plante comprend en outre une enzyme malique à NADP et une pyruvate déshydrogénase.

12. Cellule de plante selon l'une quelconque des revendications 1 à 11, dans laquelle ladite cellule de plante a une efficacité accrue d'assimilation du CO₂ en comparaison avec une cellule de plante dépourvue des activités enzymatiques destinées à convertir le glycolate en malate.

13. Cellule de plante selon l'une quelconque des revendications 1 à 12, dans laquelle ladite cellule de plante est une cellule de plante en C₃.

14. Plante comprenant la cellule végétale selon l'une quelconque des revendications 1 à 13.

15. Plante selon la revendication 14, dans laquelle ladite plante a une assimilation accrue du CO₂ en comparaison avec une plante dépourvue d'une cellule végétale selon l'une quelconque des revendications 1 à 13.

16. Plante selon la revendication 14 ou la 15, dans laquelle ladite plante a une photo-respiration atténuée en comparaison avec une plante dépourvue d'une cellule végétale selon l'une quelconque des revendications 1 à 13.

17. Plante selon l'une quelconque des revendications 14 à 16, dans laquelle ladite plante a une efficacité accrue d'utilisation de l'eau en comparaison avec une plante dépourvue d'une cellule végétale selon l'une quelconque des revendications 1 à 13.

18. Plante selon l'une quelconque des revendications 14 à 17, dans laquelle ladite plante produit un nombre accru de feuilles en comparaison avec une plante dépourvue d'une cellule végétale selon l'une quelconque des revendications 1 à 13.

19. Plante selon l'une quelconque des revendications 14 à 18, dans laquelle ladite plante a un rendement accru en comparaison avec une plante dépourvue d'une cellule végétale selon l'une quelconque des revendications 1 à 13.

20. Graine que l'on peut obtenir à partir d'une plante selon l'une quelconque des revendications 14 à 19, dans laquelle ladite graine est capable de générer une plante selon l'une quelconque des revendications 14 à 19.

21. Procédé de production d'une plante transgénique ou d'une cellule de plante ayant une efficacité accrue d'utilisation de l'eau en comparaison avec une plante ou une cellule de plante non-transgénique correspondante, ledit procédé comprend l'introduction, dans les chloroplastes de la plante ou de la cellule de plante transgénique, d'un premier polypeptide, d'un deuxième polypeptide et d'un troisième polypeptide, tels que définis dans l'une quelconque des revendications 1 à 10, dans laquelle ladite plante ou ladite cellule de plante transgénique est produite par transformation.

22. Procédé de production d'une plante ou d'une cellule de plante transgénique ayant un rendement accru en comparaison avec une plante ou une cellule de plante non-transgénique correspondante, ledit procédé comprend l'introduction, dans les chloroplastes de la plante ou de la cellule de plante transgénique, d'un premier polypeptide, d'un deuxième polypeptide et d'un troisième polypeptide, tels que définis dans l'une quelconque des revendications 1 à 10, dans laquelle ladite plante ou ladite cellule de plante transgénique est produite par transformation.

23. Procédé selon la revendication 21 ou la 22, dans laquelle ledit procédé comprend les étapes consistant à :
a. introduire au moins un polynucléotide hétérologue, qui code pour lesdits polypeptides dans la plante ou la cellule de plante ; et
b. exprimer lesdits polypeptides à partir dudit au moins un polynucléotide.

24. Polynucléotide comprenant, en combinaison, un acide nucléique tel que défini dans la revendication 2, un acide nucléique tel que défini dans la revendication 3, et un acide nucléique tel que défini dans la revendication 4.

25. Vecteur comprenant le polynucléotide selon la revendication 24.

26. Vecteur selon la revendication 25, dans laquelle ledit vecteur est un vecteur d'expression.

27. Composition comprenant un premier polynucléotide tel que défini dans la revendication 2, un deuxième polynucléotide tel que défini dans la revendication 3, et un troisième polynucléotide tel que défini dans la revendication 4.

28. Utilisation du polynucléotide selon la revendication 24, du vecteur selon la revendication 25, ou de la composition selon la revendication 27, pour conférer à une plante ou à une cellule de plante une efficacité accrue d'utilisation de l'eau.

29. Utilisation du polynucléotide selon la revendication 24, du vecteur selon la revendication 25, ou de la composition selon la revendication 27, pour conférer à une plante ou à une cellule de plante un rendement accru.
